# FASCICULE DE BREVET EUROPEEN

(11) **EP 1 032 695 B1**
(45) Date de publication et mention de la délivrance du brevet: **23.04.2008**
(21) Numéro de dépôt: 98955679.0
(22) Date de dépôt: 17.11.1998
(51) Int. Cl.: C12N 15/86, C12N 5/10

(54) **VECTEURS ADENOVIRAUX ET METHODE DE REDUCTION DES EVENEMENTS DE RECOMBINAISON HOMOLOGUE**
ADENOVIRALE VEKTOREN UND EINE METHODE ZUR REDUKTION VON HOMOLOGER REKOMBINATION
ADENOVIRUS VECTORS AND METHOD FOR REDUCING HOMOLOGOUS RECOMBINATION PHENOMENA

(30) Priorité: 17.11.1997 FR 9714383
(43) Date de publication de la demande: 06.09.2000
(73) Titulaire: CENTELION, 94400 Vitry Sur Seine (FR)
(72) Inventeur: CROUZET, Joël, F-92330 Sceaux (FR); ROBERT, Jean-Jacques, F-92330 Sceaux (FR); VIGNE, Emmanuelle, F-94200 Ivry sur Seine (FR); YEH, Patrice, F-91190 Gif sur Yvette (FR)
(74) Mandataire: Bouvet, Philippe
(86) Numéro de dépôt international: PCT/FR1998/002453
(87) Numéro de publication internationale: WO 1999/025861

(56) Documents cités:
- WO-A-96/13596
- WO-A-96/30534
- WO-A-97/00326
- MORGENSTERN J P. AND LAND H.: "Advanced mammalian gene transfer: high titre retroviral vectors with multiple drug selection markers and a complementary helper-free packaging line." NUCLEIC ACIDS RESEARCH, vol. 18, no. 12, 1990, pages 3587-3596, XP002073969
- KROUGLIAK V ET AL: "DEVELOPMENT OF CELL LINES CAPABLE OF COMPLEMENTING E1, E4, AND PROTEIN IX DEFECTIVE ADENOVIRUS TYPE 5 MUTANTS" HUMAN GENE THERAPY, vol. 6, no. 12, 1 décembre 1995, pages 1575-1586, XP000575816

## Description

La présente invention concerne une méthode permettant de réduire la fréquence des événements de recombinaison homologue intra- ou intermoléculaire entre l'acide nucléique chromosomique de la cellule comprenant la région E1 d'un génome d'adénovirus ainsi qu'une région flanquante, et un acide nucléique extra-chromosomique comprenant un génome d'adénovirus défectif pour la région E1, caractérisé en ce que la séquence de l'un au moins des deux acides nucléiques est une séquence codante et est dégénérée. Elle concerne également l'application de cette méthode dans des procédés de préparation d'acides nucléiques tels que des plasmides ou vecteurs viraux. L'invention concerne également de nouvelles constructions virales.

La recombinaison entre acides nucléiques est un phénomène bien connu de la biologie moléculaire. La recombinaison est un mécanisme moléculaire par lequel de nouvelles combinaisons de matériel génétique sont générées, contribuant à l'évolution darwinienne en fournissant une source de matériel pour la sélection naturelle. La recombinaison génétique nécessitant une forte homologie de séquence entre les acides nucléiques participant est généralement désignée recombinaison homologue. Lors de recombinaisons homologues, un échange d'information génétique se produit entre deux régions d'un acide nucléique, échange qui peut être réciproque ("crossing over") ou non réciproque (conversion).

Lors de la méiose, la recombinaison homologue est responsable du réassortiment de l'information génétique, et joue un role important dans la ségrégation correcte des chromosomes. Lors de la mitose, la recombinaison homologue participe à la réparation de l'ADN. Elle peut introduire des réarrangements génomiques, tels que des délétions et des duplications lorsqu'elle implique des régions homologues dispersées, ou aussi des contractions ou expansions lorsqu'il s'agit de séquences répétées en tandem.

Le mécanisme par lequel se produit la recombinaison homologue a été en partie élucidé. Ainsi, chez les bactéries, la recombinaison homologue commence par une étape faisant intervenir un bout simple-brin (Holliday, 1964; Meselson, 1975). Chez les eucaryotes, en revanche, la plupart des résultats suggèrent un mécanisme de cassure double-brin (DSB "double-strand break") (Szostak et al, 1983). Les DSB semblent être à l'origine de deux mécanismes principaux de recombinaison homologue : l'un, conservatif, selon lequel toutes les séquences des acides nucléiques participant à la recombinaison se retrouvent présentés dans les produits de la recombinaison (Szostak et al), l'autre, non conservatif, au cours duquel certaines séquences sont perdues. Dans les cellules somatiques mammifères, la majorité des recombinaisons homologues par DSB semblent se faire selon un processus non-conservatif (Lin et al. 1990, Jeong-Yu, 1992).

Avec le développement constant de la biotechnologie, une exploitation de plus en plus grande de l'ADN est effectuée : production de protéines recombinantes, création d'animaux transgéniques, thérapies génique et cellulaire, etc. Dans ces différents domaines, la réalisation d'événements de recombinaison non contrôlés peut constituer dans certains cas un inconvénient.

Ainsi, lors de la production de protéines recombinantes, des événements de recombinaison dans le plasmide d'expression (recombinaison intramoléculaire) peuvent par exemple conduire à l'excision de la cassette d'expression du transgène, et ainsi à une perte de l'expression. Des événements de recombinaison peuvent également être à l'origine de l'excision d'une cassette d'expression stablement intégrée dans le génome d'une cellule hôte productrice, et ainsi induire une perte de stabilité.

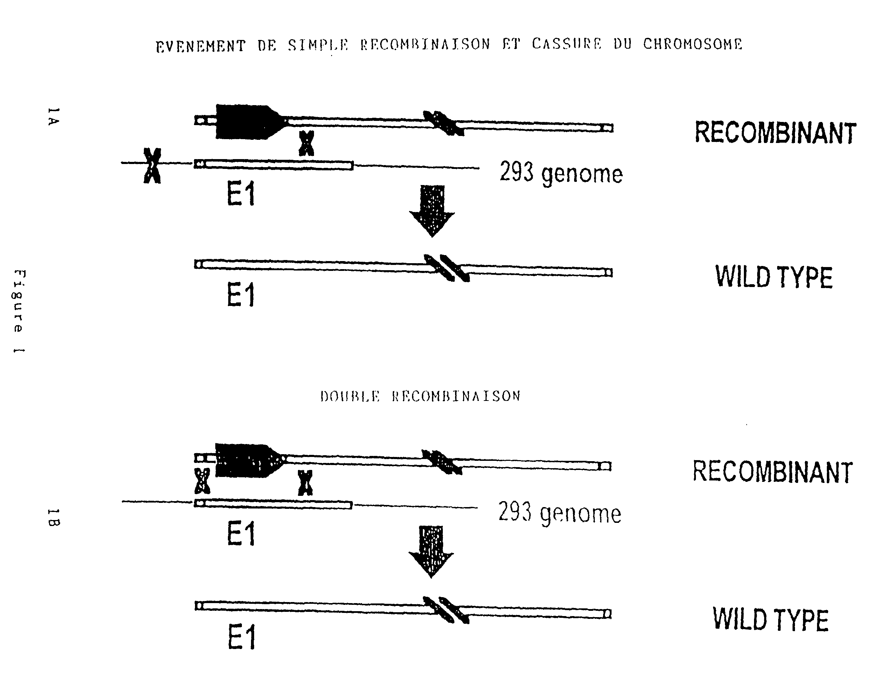

Un autre exemple d'effets indésirables liés à la réalisation d'événements de recombinaison homologue est susceptible de se produire lors de la construction et de la production de vecteurs, en particulier de vecteurs viraux. Les vecteurs viraux (adénovirus, rétrovirus, virus adéno-associé, virus de l'herpès, etc) constituent des moyens particulièrement efficaces pour le transfert d'acides nucléiques dans des cellules, in vitro, ex vivo ou in vivo. Pour la construction de vecteurs viraux défectifs, les régions essentielles à la réplication du virus sauvage sont généralement délétées du génome, et remplacées par l'acide nucléique d'intérêt. Pour produire et amplifier ces virus, il est donc nécessaire de fournir en trans les fonctions de complémentation (soit sur un plasmide, soit sous forme intégrée au génome de la cellule productrice, soit par un virus auxiliaire). Or, dans certains cas, des événements de recombinaison homologue surviennent entre le génome viral défectif et les fonctions de complémentation, reconstituant des particules virales réplicatives. Ainsi, les vecteurs dérivés des adénovirus sont généralement produits dans une lignée de complémentation (lignée 293 ou dérivée) dans laquelle une partie du génome de l'adénovirus a été intégrée. Plus précisément, la lignée 293 contient l'extrémité gauche (environ 11-12%) du génome de l'adénovirus sérotype 5 (Ad5), comprenant l'ITR gauche, la région d'encapsidation et la région E1, incluant E1a, E1b, et une partie des régions codant pour la protéine pIX et IVa2. Cette lignée est capable de trans-complémenter des adénovirus recombinants défectifs pour la région E1, c'est-à-dire dépourvus de tout ou partie de la région E1, nécessaire à la réplication. Néanmoins, il existe des zones d'homologie entre la région de l'adénovirus intégrée dans le génome de la lignée et l'ADN du virus recombinant que l'on souhaite produire. De ce fait, au cours de la production, différents événements de recombinaison peuvent se produire, générant des particules virales réplicatives, notamment des adénovirus de type E1+. Comme indiqué sur la figure 1, il peut s'agir d'un événement simple de recombinaison suivi d'une cassure du chromosome (figure 1A), ou d'une double recombinaison (figure 1B). Ces deux types de modification conduisent à remplacer la partie gauche de l'ADN recombinant, dépourvue d'une région E1 fonctionnelle, par la partie correspondante présente dans le génome de la cellule, qui porte une copie fonctionnelle de la région E1. Par ailleurs, compte tenu des titres élevés de vecteur recombinant produits par la lignée 293, la probabilité que ces événements de recombinaison aient lieu est élevée. De fait, il a été constaté que de nombreux lots de vecteurs adénoviraux recombinants défectifs étaient contaminés par des particules virales réplicatives.

La présence de particules réplicatives dans les lots de virus constitue un inconvénient important pour des applications pour le transfert de gènes in vitro ou in vivo (risques de propagation virale et de dissémination incontrôlée).

Le même type de problématique existe pour la génération de rétrovirus défectifs. Ainsi, les rétrovirus défectifs construits sont généralement délétés des régions virales codantes (gag, pol et env), qui sont apportées en trans par la lignée de production. Ici encore, pour certaines lignées décrites, des zones de recouvrement existent entre le génome du rétrovirus défectif et les fonctions de complémentation portées par la cellules. C'est le cas en particulier des cellules PA317, Psi2, etc. Des événements de recombinaison homologue sont donc susceptibles de se produire au niveau de ces zones, générant des particules réplicatives.

La présente invention est relative à un procédé permettant de réduire la fréquence des événements de recombinaison homologue intra- ou intermoléculaire entre l'acide nucléique chromosomique de la cellule comprenant la région E1 d'un génome d'adénovirus ainsi qu'une région flanquante, et un acide nucléique extra-chromosomique comprenant un génome d'adénovirus défectif pour la région E1, caractérisé en ce que la séquence de l'un au moins des deux acides nucléiques est une séquence codante et est dégénérée.

Pour réduire les événements de recombinaison homologue, l'art antérieur enseigne différentes approches, qui reposent toutes sur un même principe : remplacer ou supprimer les régions d'homologie. Ainsi, certains plasmides permettant l'expression de gènes d'intérêt portent des régions homologues au génome de la cellule hôte. Il peut s'agir en particulier d'une région promoteur, d'un gène marqueur ou d'une origine de réplication. Pour réduire les risques de recombinaison, il a été proposé jusqu'à présent de substituer ces régions par d'autres, non homologues (promoteur différent, etc). D'autre part, pour limiter les risques de recombinaison dans les procédés de production de vecteurs viraux, il a été suggéré d'éliminer les séquences homologues entre les gènes de complémentation et le génome viral défectif.

Ainsi, la demande de brevet WO97/00326 décrit une lignée cellulaire de production d'adénovirus, désignée PER, comprenant une unité restreinte du génome adénoviral portant la région E1. Avec cette Lignée, les séquences flanquantes homologues au génome du virus défectif sont réduites, ce qui permet de limiter les risques de recombinaison homologue entre ceux-ci. De la même façon, la demande WO95/11984 décrit la construction d'un adénovirus recombinant portant une délétion de la région E1 étendue à une portion du gène pIX. Dans ce cas, ce n'est plus la région de complémentation qui est modifiée (réduite), mais la délétion portée dans le génome défectif, qui est étendue. Le résultat est également une diminution des risques de recombinaison, même si des régions d'homologie subsistent.

Cependant, si ces approches permettent de réduire les risques de recombinaison entre la cellule et le génome viral, et donc les risques de produire des lots de virus contaminés par des particules réplicatives, elles ne permettent pas de les éliminer totalement et/ou nécessitent la construction et donc la validation de nouvelles lignées cellulaires, ce qui est très lourd. Par ailleurs, la substitution de régions par d'autres n'est pas forcément satisfaisante, notamment en terme d'efficacité.

La présente demande décrit un nouveau procédé permettant de réduire les événements de recombinaison homologue inter- ou intramoléculaires. L'invention décrit également l'application de ce procédé à la production de virus défectifs non contaminés par des particules réplicatives. L'invention décrit également de nouvelles constructions virales capables d'être amplifiées dans les lignées de production existantes les plus efficaces sur le plan du titre, avec des risques de génération de particules réplicatives fortement réduits.

L'étape initiale et importante de la recombinaison homologue est la reconnaissance entre les deux acides nucléiques partenaires (recombinaison intermoléculaire) ou entre deux régions d'un acide nucléique (recombinaison intramoléculaire). Cette étape est le résultat d'une interaction directe entre les deux régions homologues. La recombinaison homologue entre deux acides nucléiques est donc basée sur l'existence d'une identité, ou d'une forte homologie de séquence entre deux régions de ces acides nucléiques, et elle est généralement dépendante de deux facteurs : le degré d'homologie et la longueur de l'homologie (i.e. des régions homologues portées par le ou les deux acides nucléiques). Par ailleurs, la fréquence de recombinaison homologue peut également être influencée par certaines régions particulières des acides nucléiques. Ainsi, il a été observé que certaines régions étaient capables de recombiner avec une fréquence plus élevée que la fréquence moyenne. Il a par ailleurs été déterminé que ces variations de fréquence localisées pouvaient être dues à des séquences particulières telles que des sites CHI chez *E. coli* ou des sites M26 chez *S. pombe* (Gangloff et al., 1994).

Contrairement aux stratégies proposées dans l'art antérieur, le procédé de l'invention ne passe pas par une suppression des zones d'homologie entre les acides nucléiques partenaires. Le procédé de l'invention repose de manière originale sur la modification de la séquence de l'un au moins des deux acides nucléiques partenaires de la recombinaison, de manière à réduire l'homologie existant entre ces acides nucléiques.

Un premier objet de l'invention concerne donc un procédé permettant de réduire la fréquence des événements de recombinaison homologue intra- ou intermoléculaire entre acides nucléiques caractérisé en ce que la séquence de l'un au moins des acides nucléiques partenaires de la recombinaison est dégénérée de manière à réduire l'homologie avec le ou les autre(s) partenaires.

On entend au sens de l'invention par "réduction de la fréquence des événements de recombinaison homologue" entre acides nucléiques tout abaissement de ladite fréquence, par rapport à la fréquence observée avec les acides nucléiques correspondants non modifiés. Cette réduction peut être mesurée aisément par des tests classiques connus de l'homme du métier (en particulier par des tests de "marker rescue" ou par des tests de génération de particules virales réplicatives). Avantageusement, le terme "réduction" s'entend d'une baisse significative de la fréquence de recombinaison homologue, de préférence d'une unité logarithmique au moins.

On entend par recombinaison homologue intermoléculaire une recombinaison homologue entre deux acides nucléiques (ou entre deux régions de deux acides nucléiques) distincts. On entend par recombinaison homologue intramoléculaire une recombinaison homologue entre deux régions d'un même acide nucléique. Par ailleurs, les acides nucléiques partenaires de la recombinaison homologue peuvent être des acides nucléiques extrachromosomiques, chromosomiques, ou une combinaison des deux (i.e. un acide nucléique chromosomique et un acide nucléique extrachromosomique). Les acides nucléiques extrachromosomiques peuvent être des plasmides, vecteurs, épisomes, génomes viraux, etc.

Le procédé de l'invention est particulièrement adapté pour réduire la fréquence des événements de recombinaison homologue intermoléculaire entre un acide nucléique chromosomique et un acide nucléique extra-chromosomique.

Le procédé de l'invention implique généralement les étapes suivantes:
(i) identification de la ou des régions responsables de la recombinaison homologue
(ii) modification de cette ou ces régions
(iii) vérification de la séquence
(iv) synthèse de la séquence modifiée (désignée syngen)
(v) remplacement de la séquence originelle par le syngen

(i) L'identification des régions responsables de la recombinaison homologue entre acides nucléiques est réalisée par toute méthode connue. En particulier, dès lors qu'un événement de recombinaison est observé, les régions qui en sont responsables peuvent être recherchées par analyse de séquence : recherche de régions homologues entre les acides nucléiques (intermoléculaire) ou au sein de l'acide nucléique (intramoléculaire).
   Lorsque les séquences impliquées dans la recombinaison sont identifiées, une région est définie, comprenant tout ou une partie de ces séquences, qui est utilisée pour l'étape (ii) ci-après, c'est-à-dire la modification.
(ii) Modification de la séquence
   Il est généralement admis que l'homologie doit être très forte sur une région suffisamment longue pour qu'un événement de recombinaison puisse avoir lieu à une fréquence significative. En particulier, les données de la littérature suggèrent qu'une région d'homologie parfaite d'une longueur au moins égale à environ 200 pb est requise pour la réalisation de tels événements. En effet, même si des recombinaisons peuvent se produire sur des régions plus réduites, leur fréquence est beaucoup plus faible et non régulière. Par ailleurs, sur une telle région dont l'homologie est réduite de 19%, il semble que la fréquence de recombinaison soit réduite d'un facteur 1000 (Waldman et Liskay, 1987).
   La séquence peut être modifiée de différentes manières. S'agissant de séquence codante, des modifications peuvent être apportées fondées sur la dégénérescence du code génétique. Ainsi, la séquence est perturbée, et donc l'homologie est réduite, mais le produit d'expression est le même.
   L'invention réside donc en particulier dans une modification de la séquence de manière à empêcher l'appariement entre les deux régions homologues. La modification permet de diminuer la longueur et le degré d'homologie entre les deux régions concernées.
   Avantageusement, dans le procédé de l'invention, la séquence de l'acide nucléique est dégénérée, au niveau de la région impliquée dans la recombinaison homologue, à raison de 1 paire de bases au moins toutes les 20 paires de bases. Plus préférentiellement, elle est dégénérée à raison de 1 paire de bases au moins toutes les 10 paires de bases.
   Selon une variante particulière de l'invention, la séquence est dégénérée sur toutes les positions possibles.
   La dégénérescence de la séquence selon l'invention est avantageusement réalisée en fonction de l'usage des codons de la cellule ou l'organisme chez lequel l'acide nucléique doit être utilisé. Dans le cas d'un vecteur viral dont la production est réalisée dans une lignée de cellules humaines, il est particulièrement intéressant de dégénérer les séquences en favorisant l'usage préféré des codons chez l'homme lorsque ce choix est possible (voir exemples).
   Par ailleurs, des modifications supplémentaires peuvent être apportées à la séquence d'acide nucléique. Ainsi, dans les régions non codantes, il est possible de réduire la taille de certains éléments (séquences régulatrices de l'expression, promoteurs) ou de modifier ces éléments ou de substituer certains autres éléments par des régions hétérologues .
   Selon une variante particulière de l'invention, et lorsque la zone d'homologie s'étend sur plusieurs gènes, il est possible de réduire la zone d'homologie d'une part, en dégénérant la séquence de un ou plusieurs gènes et, d'autre part, en modifiant la position génomique de certains gènes présents dans la zone d'homologie c'est à dire en plaçant ces gènes au sein du génome adénoviral et dans une position génomique autre que leur position d'origine. La séquence des gènes dont la position génomique est modifiée peut en outre être dégénérée. De manière préférée, seule la séquence des gènes qui ne sont pas déplacés est dégénérée.
(iii) Vérification de la séquence
   La vérification est réalisée par des méthodes informatiques, permettant de détecter la présence d'éléments de régulation, structures secondaires, etc, susceptibles d'interférer avec l'activité du syngen. Voir exemples.
(iv) Synthèse du syngen
   Le syngen peut être synthétisé par toute technique connue de l'homme du métier, et notamment en utilisant des synthétiseurs d'acides nucléiques.
(v) remplacement de la séquence originelle par le syngen
   Le syngen, une fois synthétisé, est ensuite introduit dans l'acide nucléique en remplacement de la séquence originelle. Cette étape peut également être réalisée selon les techniques de biologie moléculaire classique bien connues de l'homme du métier.

L'une des applications du procédé de l'invention réside dans la production de vecteurs, notamment de vecteurs viraux, dépourvus de particules virales compétentes pour la réplication (RCV). A cet égard, le procédé de l'invention vise plus particulièrement à réduire la fréquence des événements de recombinaison homologue entre un acide nucléique chromosomique codant pour des fonctions de complémentation d'un virus défectif et un acide nucléique extra-chromosomique comprenant le génome dudit virus défectif.

Le virus concerné peut être avantageusement un adénovirus, un rétrovirus, un virus adéno-associé (AAV) ou encore un virus de l'herpès. Il s'agit plus préférentiellement d'un adénovirus.

Ainsi, un mode de réalisation particulier de l'invention consiste en un procédé de réduction de la fréquence des événements de recombinaison homologue entre un acide nucléique chromosomique comprenant la région E1 d'un génome d'adénovirus ainsi qu'une région flanquante et un acide nucléique extra-chromosomique comprenant un génome d'adénovirus défectif pour la région E1.

Avantageusement, dans ce procédé particulier, la séquence dégénérée selon l'invention comprend le gène pIX du génome de l'adénovirus défectif pour la région E1. Encore plus préférentiellement, la séquence dégénérée comprend les gènes pIX et IVa2 du génome de l'adénovirus.

Selon une autre variante de réalisation, la séquence dégénérée comprend le gène pIX du génome de l'adénovirus, et la séquence du gène Iva2 est déplacée de son locus naturel vers la région E4.

Le procédé de l'invention est particulièrement adapté à la production d'adénovirus recombinants défectifs pour la région E1 dans la lignée cellulaire 293 ou dans une lignée cellulaire dérivée.

Comme indiqué ci-avant, la lignée cellulaire 293 contient dans son génome la partie gauche du génome de l'adénovirus comprenant notamment la région E1 et une région flanquante située en aval (3') de la région E1 portant notamment le gène pIX et une partie du gène IVa2. C'est précisément au niveau de cette région flanquante que se produisent des recombinaisons homologues générant des Adénovirus compétents pour la réplication (ACR). On entend au sens de l'invention par "lignée cellulaire dérivée" une lignée cellulaire portant la région E1 de l'adénovirus et une région flanquante susceptible de donner lieu à une recombinaison avec le virus défectif. Il peut s'agir d'une région plus courte que celle présente dans 293 (limitée à pIX par exemple) ou d'une région plus longue. Une lignée dérivée peut également être une lignée construite à partir des cellules 293, par introduction de séquences de complémentation supplémentaires (telles que E4).

A cet égard, l'invention concerne également un procédé de préparation d'adénovirus recombinants défectifs par introduction, dans une cellule de la lignée 293 ou dans une cellule dérivée, du génome dudit adénovirus recombinant défectif, caractérisé en ce que ledit génome porte :
- une délétion de la région E1
- une dégénérescence dans les gènes pIX et/ou IVa2.

L'invention concerne en outre tout vecteur viral dont le génome comprend une région au moins dont la séquence est dégénérée. Il peut s'agir notamment de rétrovirus (portant des séquences dégénérées dans les gènes gag, pol et ou env) d'AAV (portant des séquences dégénérées dans les gènes rep et/ou cap) ou également d'adénovirus.

Il s'agit avantageusement d'adénovirus dont le génome porte un gène pIX dégénéré. De préférence, la séquence dégénérée du gène pIX est la séquence ID N°1 ou la séquence ID N° 4. De préférence encore, l'adénovirus comporte en outre une modification de la position génomique du gène Iva2. Ce gène est avantageusement positionné au niveau de la région E4.

Selon un autre mode de réalisation, il s'agit d'un adénovirus dont les gènes pIX et IVa2 sont dégénérés. De préférence, la séquence naturelle du gène pIX est remplacée par la séquence SEQ ID N° 1 ou la séquence SEQ ID N°4 et la séquence dégénérée du gène IVa2 est la séquence SEQ ID N°2 ou la séquence SEQ ID N°5. De préférence, la séquence naturelle des gènes pIX et IVa2 est remplacée par la séquence SEQ ID N° 3.

Selon un autre mode de réalisation, il s'agit d'un adénovirus dont les gènes pIX et IVa2 sont dégénérés et qui comporte en outre des modifications de la séquence du promoteur du gène pIX et /ou un remplacement de la séquence de polyadénylation de ces gènes. De préférence, il s'agit d'un adénovirus comportant la séquence SEQ ID N°8.

L'adénovirus porte en outre avantageusement au moins une délétion de la région E1. Plus préférentiellement, les adénovirus sont défectifs pour tout ou partie des régions E1 et E3 au moins. Il peut s'agir également d'adénovirus recombinants défectifs pour les régions E1 et E4, en tout ou partie, et éventuellement pour la région E3. Par ailleurs, cet adénovirus peut être de différents sérotypes. Concernant les adénovirus d'origine humaine, on peut citer préférentiellement ceux classés dans le groupe C.

Plus préférentiellement encore, parmi les différents sérotypes d'adénovirus humain, on préfère utiliser dans le cadre de la présente invention les adénovirus de type 2 ou 5 (Ad 2 ou Ad 5). Parmi les différents adénovirus d'origine animale, on préfère utiliser dans le cadre de l'invention des adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914 incorporée à la présente par référence. Les stratégies de construction des adénovirus, ainsi que les sites utilisables pour l'introduction de gènes d'intérêt dans ces vecteurs sont décrits en détail dans l'art antérieur, et notamment WO95/02697, WO96/10088, WO96/13596 ou encore WO96/22378.

Selon un mode particulièrement avantageux, dans les adénovirus recombinants de la présente invention, la région E1 est inactivée par délétion d'un fragment PvuII-BgIII allant du nucléotide 454 au nucléotide 3328, sur la séquence de l'adénovirus Ad5. Cette séquence est accessible dans la littérature et également sur base de données (voir notamment Genebank n° M73260). Dans un autre mode de réalisation préféré, la région E1 est inactivée par délétion d'un fragment HinfII-Sau3A allant du nucléotide 382 au nucléotide 3446.

Avantageusement, les adénovirus recombinants de l'invention comportent en outre une séquence d'acides nucléiques hétérologue dont le transfert et/ou l'expression dans une cellule, un organe ou un organisme est recherché.

En particulier, la séquence d'ADN hétérologue peut comporter un ou plusieurs gènes thérapeutiques et/ou un ou plusieurs gènes codant pour des peptides antigéniques.

Les gènes thérapeutiques qui peuvent ainsi être transférés sont tout gène dont la transcription et éventuellement la traduction dans la cellule cible génèrent des produits ayant un effet thérapeutique.

Il peut s'agir en particulier de gènes codant pour des produits protéiques ayant un effet thérapeutique. Le produit protéique ainsi codé peut être une protéine, un peptide, un acide aminé, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule lui permettant de lutter contre une pathologie.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, VEGF, aFGF, bFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc, le gène codant pour la protéine GAX, les gènes suicides : thymidine kinase, cytosine désaminase, etc., des gènes codant pour des anticorps simple chaîne (scFv)

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Comme indiqué plus haut, la séquence d'ADN hétérologue peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, la séquence d'acides nucléiques hétérologue comprend également une région promotrice de la transcription fonctionnelle dans la cellule infectée. Il peut s'agir d'une région promotrice naturellement responsable de l'expression du gène considéré lorsque celle-ci est susceptible de fonctionner dans la cellule infectée. Il peut également s'agir de régions d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces régions promotrices peuvent être modifiées par addition de séquences d'activation, de régulation, ou permettant une expression tissu-spécifique ou majoritaire. Par ailleurs, lorsque l'acide nucléique hétérologue ne comporte pas de séquences promotrices, il peut être inséré dans le génome du virus défectif en aval d'une telle séquence.

Par ailleurs, la séquence d'acides nucléiques hétérologue peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Toujours dans un mode particulierement avantageux, les vecteurs de l'invention possèdent en outre un gène E3 fonctionnel sous controle d'un promoteur hétérologue. Plus préférentiellement, les vecteurs possèdent une partie du gène E3 permettant l'expression de la protéine gp19K. Cette protéine permet en effet d'éviter que le vecteur adénovirus fasse l'objet d'une réaction immunitaire qui (i) limiterait son action et (ii) pourrait avoir des effets secondaires indésirables.

Ces vecteurs recombinants peuvent être utilisés pour le transfert d'acides nucléiques dans les cellules in vitro, ex vivo ou in vivo.

La présente invention concerne également toute composition pharmaceutique comprenant un ou plusieurs adénovirus recombinants tels que décrits précédemment. Les compositions pharmaceutiques de l'invention peuvent être formulées en vue d'une administration par voie topique, orale, parentérale, intranasale, intraveineuse, intramusculaire, sous-cutanée, intraoculaire, transdermique, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 5 jours, du nombre de plages de cellules infectées. Les techniques de détermination du titre pfu d'une solution virale sont bien documentées dans la littérature.

La présente invention sera décrite plus en détail à l'aide des exemples qui suivent qui doivent être considérés comme illustratifs et non limitatifs.

### Légende des Figures

Figure 1 : Evènements de recombinaison entre l'adénovirus et la lignée 293
Figure 2 : Structure schématique du syngen
Figure 3 : carte de restriction du plasmide pJJ 105
Figure 4 : carte de restriction du plasmide pJJ 110
Figure 5 : carte de restriction du plasmide pJJ 206
Figure 6 : construction du virus défectif portant le syngen en remplacement de la région naturelle.
Figure 7 : Structure et carte de restriction des oligonucléotides Oligosyngen I et Oligosyngen II.
Figure 8 : Séquence nucléotidique naturelle et séquence dégénérée du gène pIX (SEQ ID N°1), et séquence correspondante d'acides aminés.
Figure 9 : Séquence nucléotidique naturelle et séquence dégénérée du gène Iva2 (SEQ ID N°2), et séquence correspondante d'acides aminés.
Figure 10 : Séquence nucléotidique du syngen # 1 (SEQ ID N°3)

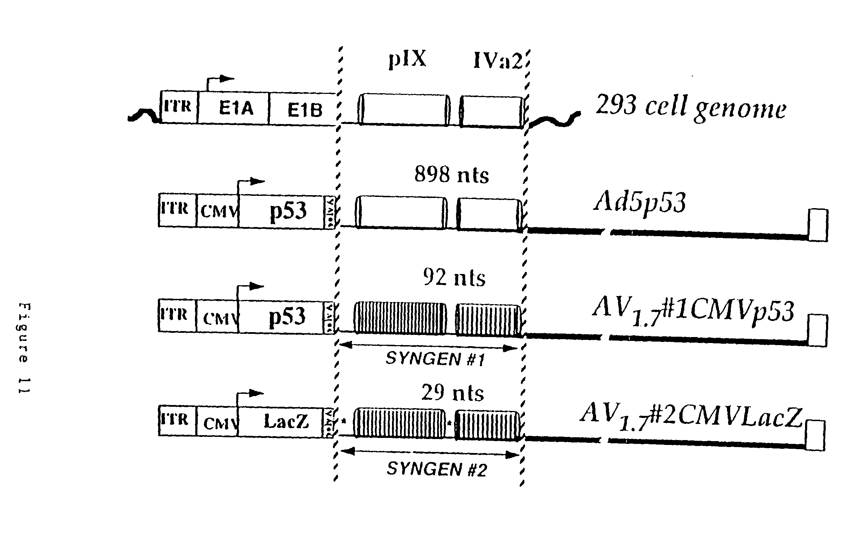

Figure 11 : représentation schématique des homologies entre le génome de la cellule 293 et des vecteurs Ad5 recombinants. Le vecteur Ad5p53 présente une séquence d'homologie continue de 898 nucléotides avec le génome de la cellules 293 en aval de la région E1. L'introduction de mutations ponctuelles réduit la longueur maximale d'homologie continue à 92 nucléotides dans le cas du syngen # 1 (AV 1.7 # 1 CMV p53) et à 29 nucléotides dans le cas du syngen # 2 (AV 1.7 # 2 CMV lac Z).
Figure 12 : Détection de ACR pour des vecteurs Ad5p53 et AV1.7 #1 p53 après 7 amplifications successives dans des cellules 293.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides pIC-20R (Invitrogen) et pBS (Stratagen) sont d'origine commerciale.

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Lignées cellulaires utilisées.

- Lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %).

### Exemple 1 : Recombinaison homologue dans l'adénovirus

L'observation que le génome de l'adénovirus peut subir des recombinaisons génétiques a été rapportée il y a plus de 25 ans (Williams et Ustacelibi, 1971). Toutefois, les mécanismes soutenant ces recombinaisons sont assez peu élucidés. Il a été proposé que la recombinaison et la réplication puissent être intimement liées, et certaines données sont en faveur d'un modèle de recombinaison dans lequel la réplication produirait les substrats spécifiques nécessaires pour les recombinaisons fréquentes observées lors de l'infection par l'adénovirus. L'une des questions est de savoir si des protéines virales ou cellulaires participent à la formation des intermédiaires de la recombinaison et dans leur résolution en produits recombinants finals. A cet égard, aucun des gènes viraux testés n'a été montré comme impliqué dans les événements de recombinaison : E1b, E4, E1a, E3 (Epstein, 1991; Weinberg et al., 1986; Young, 1995). De la même manière aucune protéine cellulaire n'a pu être mise en évidence. Deux modèles ont finalement été proposés pour le mécanisme de recombinaison dans le génome adénoviral, prédisant tous deux la formation d'hétéroduplex étendus (Ahern et al., 1991; Young, 1995).

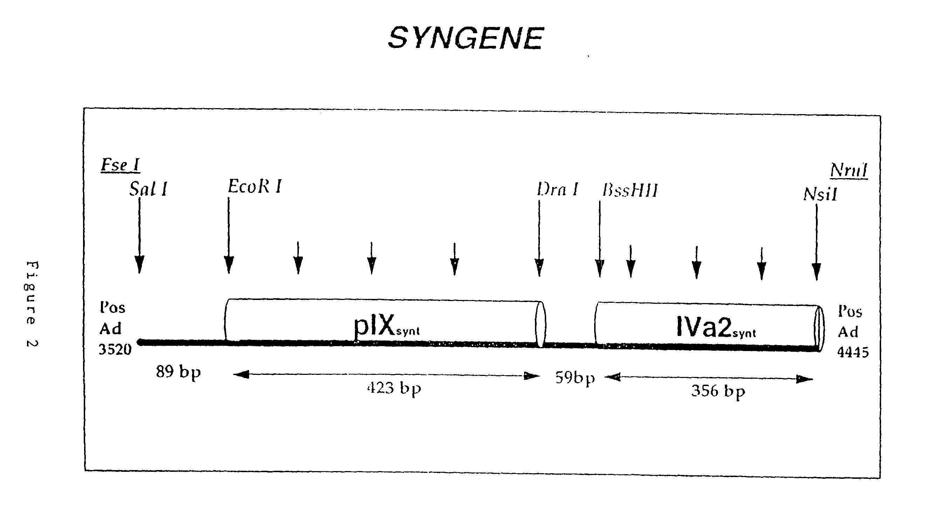

Les régions responsables de la recombinaison homologue entre le génome défectif et la lignée cellulaire ont été localisées notamment au niveau des gènes pIX et IVa2 du génome de l'adénovirus. La structure de cette région présente dans le génome défectif est représentée sur la figure 2.

Des modifications de la séquence du génome de l'adénovirus impliquée dans la recombinaison avec le génome cellulaire ont été réalisées. Plus précisément, la séquence naturelle des gènes pIX et IVa2 a été modifiée par introduction de mutations silencieuses, permettant de réduire l'homologie avec la séquence naturelle, sans affecter la structure des produits d'expression de ces gènes.

La séquence obtenue, désignée syngen, est utilisée en remplacement de la séquence naturelle correspondante de l'adénovirus, pour construire le vecteur défectif.

### 1. Identification des régions responsables de la recombinaison

Des études de cartographie ont montré que la partie du génome de fadénovirus présente dans le génome des cellules 293 correspondait à la partie gauche du génome adénoviral, et représentait environ 12% de celui-ci, soit environ 4300 pb (Aiello et al., 1979; Spector et al., 1983). La demanderesse a réalisé des expériences de cartographie supplémentaires par PCR, et a montré que la position 4420 était présente dans le génome de 293. Cette position 4420 a été choisie pour les expériences suivantes comme la limite droite de la zone d'homologie, et donc la limite droite du syngen. Pour des raisons techniques de clonage, le fragment modifié s'étendra jusqu'à la position 4445 où un site de clonage a été introduit.

L'extrémité gauche du syngen a été choisie dans les éléments de régulation de l'expression du gène pIX et, plus particulièrement, en position -56 du gène pIX. Il a en effet été montré que le promoteur du gène pIX pouvait être réduit à une région de 56 nucléotides. L'activité de cette région, contenant le site de liaison SP1 et la boite TATA est comparable à celle du fragment de 250 nucléotides (Babiss et al., 1991; Matsui et al., 1989).

La zone d'homologie a donc été déterminée comme s'étendant de la position 3520 à la position 4445 du génome du virus défectif (Voir figure 2).

### 2. Design de séquences modifiées

Une table d'usage des codons dans le génome de l'adénovirus Ad5 a été réalisée par la demanderesse et utilisée afin de déterminer l'usage optimum des codons pour l'expression des protéines virales. Cette table a été obtenue en combinant les données obtenues d'après l'analyse de 10 protéines de l'adénovirus : pIX, Hexon, pIII, pVII, 52-55k, pPol, pTP, DBP, 23K et 19k (voir table). Les résultats obtenus ont ensuite été comparés avec une table d'usage des codons humains obtenue par analyse de 1952 gènes présents dans la base Genbank en utilisant le programme GCG.

Un ordre de préférence de l'utilisation des codons a alors été établi pour modifier la séquence codante des gènes pIX et IVa2 :
- Lorsque le codon utilisé dans la séquence naturelle n'est pas le codon préféré, celui-ci est utilisé dans le syngen.
- Lorsque le codon utilisé dans la séquence naturelle est le codon préféré, c'est alors le deuxième codon préféré qui est utilisé.
- Dans certains cas, par exemple pour l'introduction d'un site de restriction, le troisième codon préféré peut être utilisé, en particulier lorsque sa fréquence est suffisamment proche de celle du deuxième codon préféré.

Le tableau ci-dessous indique la fréquence d'usage des codons chez l'Homme et chez l'adénovirus (ADV5) avec l'ordre de préférence chez l'Homme (colonne A), l'ordre de préférence pour l'adénovirus (colonne B) et le choix retenu pour la synthèse du syngen (colonne C)

**Table 1 : fréquence d'utilisation des codons**

| | | | | **ADV5** | | **Huma n** | **A** | **H** | **C** |
|---|---|---|---|---|---|---|---|---|---|
| **F** | TTT | Phe | 2,3 | 54,2% | 1,5 | 43,0% | 1 | 2 | - |
| **F** | TTC | | 1,9 | 45,8% | 2,1 | 57,0% | 2 | 1 | - |
| **L** | TTA | Leu | 0,1 | 1,4% | 0,6 | 6,0% | 6 | 6 | 6 |
| **L** | TTG | | 1,2 | 14,2% | 1,1 | 12,0% | 4 | 4 | 4 |
| | | | | | | | | | |
| **L** | CTT | Leu | 1,3 | 15,6% | 1,1 | 12,0% | 3 | 3 | 3 |
| **L** | CTC | | 1,7 | 20,5% | 1,9 | 20,0% | 2 | 2 | 2 |
| **L** | CTA | | 1,1 | 12,6% | 0,6 | 7,0% | 5 | 5 | 5 |
| **L** | CTG | | 3 | 35,6% | 4 | 43,0% | 1 | 1 | 1 |
| | | | | | | | | | |
| **I** | ATT | Ile | 1,3 | 34,6% | 1,5 | 35,0% | 2 | 2 | 2 |
| **I** | ATC | | 1,6 | 43,8% | 2,3 | 52,0% | 1 | 1 | 1 |
| **I** | ATA | | 0,8 | 21,6% | 0,7 | 14,0% | 3 | 3 | 3 |
| **M** | ATG | Met | 2,6 | 100,0% | 2,2 | 100,0% | | | |
| | | | | | | | | | |
| **V** | GTT | Val | 0,8 | 13,1% | 1 | 17,0% | 3 | 3 | 3 |
| **V** | GTC | | 1,4 | 22,2% | 1,5 | 25,0% | 2 | 2 | 2 |
| **V** | GTA | | 0,8 | 12,5% | 0,6 | 10,0% | 4 | 4 | 4 |
| **V** | GTG | | 3,3 | 52,2% | 2,9 | 48,0% | 1 | 1 | 1 |
| | | | | | | | | | |
| **Y** | TAT | Tyr | 0,8 | 21,6% | 1,2 | 41,7% | 2 | 2 | 2 |
| **Y** | TAC | | 3 | 78,4% | 1,7 | 58,3% | 1 | 1 | 1 |
| ***** | TAA | end | | | 0,1 | 23,8% | | | |
| ***** | TAG | | | | 0,1 | 16,7% | | | |
| | | | | | | | | | |
| **H** | CAT | His | 0,5 | 21,3% | 1,4 | 59,3% | 2 | 1 | - |
| **H** | CAC | | 1,7 | 78,7% | 1 | 40,7% | 1 | 2 | - |
| **Q** | CAA | Gln | 1,1 | 27,4% | 1,2 | 26,7% | 2 | 2 | 2 |
| **Q** | CAG | | 3 | 72,6% | 3,3 | 73,3% | 1 | 1 | |
| | | | | | | | | | |
| **N** | AAT | Asn | 1,2 | 24,5% | 1-,6 | 43,5% | 2 | 2 | 2 |
| **N** | AAC | | 3,6 | 75,5% | 2,1 | 56,5% | 1 | 1 | 1 |
| **K** | AAA | Lys | 1,4 | 35,2% | 2,2 | 39,8% | 2 | 2 | 2 |
| **K** | AAG | | 2,5 | 64,8% | 3,4 | 60,2% | 1 | 1 | 1 |
| | | | | | | | | | |
| **D** | GAT | Asp | 1,6 | 29,2% | 2,1 | 44,2% | 2 | 2 | 2 |
| **D** | GAC | | 3,9 | 70,8% | 2,7 | 55,8% | 1 | 1 | 1 |
| **E** | GAA | Glu | 2,4 | 37,0% | 2,8 | 41,5% | 2 | 2 | 2 |
| **E** | GAG | | 4,1 | 63,0% | 3,9 | 58,5% | 1 | 1 | 1 |
| | | | | | | | | | |
| **S** | TCT | Ser | 0,8 | 11,7% | 1,4 | 18,0% | 4 | 3 | 3 |
| **S** | TCC | | 1,7 | 25,9% | 1,7 | 23,2% | 2 | 2 | 2 |
| **S** | TCA | | 0,6 | 9,6% | 1,1 | 14,7% | 5 | 4 | 4 |
| **S** | TCG | . | 0,9 | 13,6% | 0,4 | 5,9% | 3 | 6 | 5 |
| | | | | | | | | | |
| **P** | CCT | Pro | 1,2 | 18,4% | 1,8 | 29,0% | 4 | 2 | 2 |
| **P** | CCC | | 2,7 | 39,9% | 2,1 | 33,0% | 1 | 1 | 1 |
| **P** | CCA | | 1,3 | 18,7% | 1,7 | 27,0% | 3 | 3 | 3 |
| **P** | CCG | | 1,5 | 23,0% | 0,1 | 11,0% | 2 | 4 | 4 |
| | | | | | | | | | |
| **T** | ACT | Thr | 1,1 | 17,7% | 1,3 | 23,0% | 2 | 3 | 2 |
| **T** | ACC | | 3,3 | 55,0% | 2,2 | 38,0% | 1 | 1 | 1 |
| **T** | ACA | | 0,8 | 12,8% | 1,5 | 27,0% | 4 | 2 | 4 |
| **T** | ACG | | 0.9 | 14,5% | 0,1 | 12,0% | 3 | 4 | 3 |
| | | | | | | | | | |
| **A** | GCT | Ala | 1,2 | 13,7% | 2 | 28,0% | 3 | 2 | 2 |
| **A** | GCC | | 4 | 45,7% | 2,8 | 40,0% | 1 | 1 | 1 |
| **A** | GCA | | 1,1 | 12,6% | 1,6 | 22,0% | 4 | 3 | 3 |
| **A** | GCG | | 2,4 | 27,9% | 0,7 | 10,0% | 2 | 4 | 4 |
| | | | | | | | | | |
| **C** | Cys | TGT | 0,3 | 21,5% | 1 | 41,8% | 2 | 2 | 2 |
| **C** | | TGC | 1,1 | 78,5% | 1,4 | 58,2% | 1 | 1 | 1 |
| ***** | end | TGA | | | 0,3 | 61,9% | | | |
| **W** | Trp | TGG | 1,3 | 100,0% | 1,5 | 100,0% | | | |
| | | | | | | | | | |
| **R** | Arg | CGT | 0,8 | 10,4% | 0,5 | 9,3% | 3 | 6 | 6 |
| **R** | | CGC | 4,1 | 52,4% | 1,1 | 19,4% | 1 | 3 | 1 |
| **R** | | CGA | 0,6 | 7,0% | 0,6 | 10,0% | 5 | 5 | 5 |
| **R** | | CGG | 1,1 | 13,9% | 1 | 18,6% | 2 | 4 | 2 |
| | | | | | | | | | |
| **S** | Ser | AGT | 0,4 | 6,6% | 1 | 13,6% | 6 | 5 | 6 |
| **S** | | AGC | 2,1 | 32,6% | 1,9 | 24,7% | 1 | 1 | 1 |
| **R** | Arg | AGA | 0,5 | 6,2% | 1,2 | 21,0% | 6 | 2 | 4 |
| **R** | | AGG | 0,8 | 10,2% | 1,2 | 21,7% | 4 | 1 | 3 |
| | | | | | | | | | |
| **G** | Gly | GGT | 1 | 17,7% | 1,4 | 18,3% | 4 | 4 | 4 |
| **G** | | GGC | 2,4 | 41,3% | 2,5 | 33,2% | 1 | 1 | 1 |
| **G** | | GGA | 1,3 | 21,5% | 1,9 | 25,7% | 2 | 2 | 2 |
| **G** | | GGG | 1,1 | 19,4% | 1,7 | 22,8% | 3 | 3 | 3 |

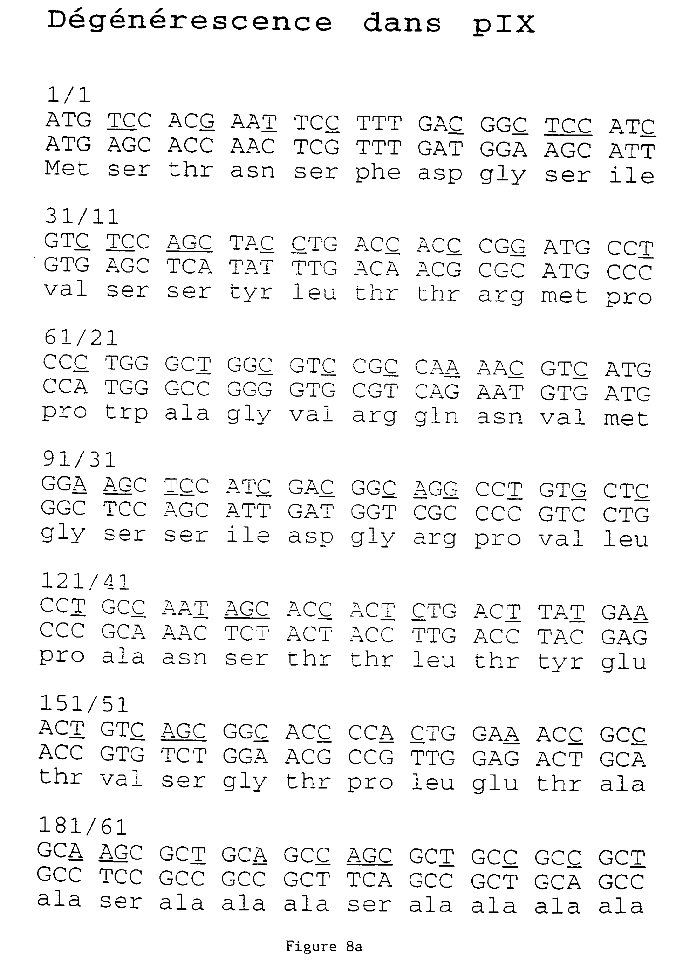

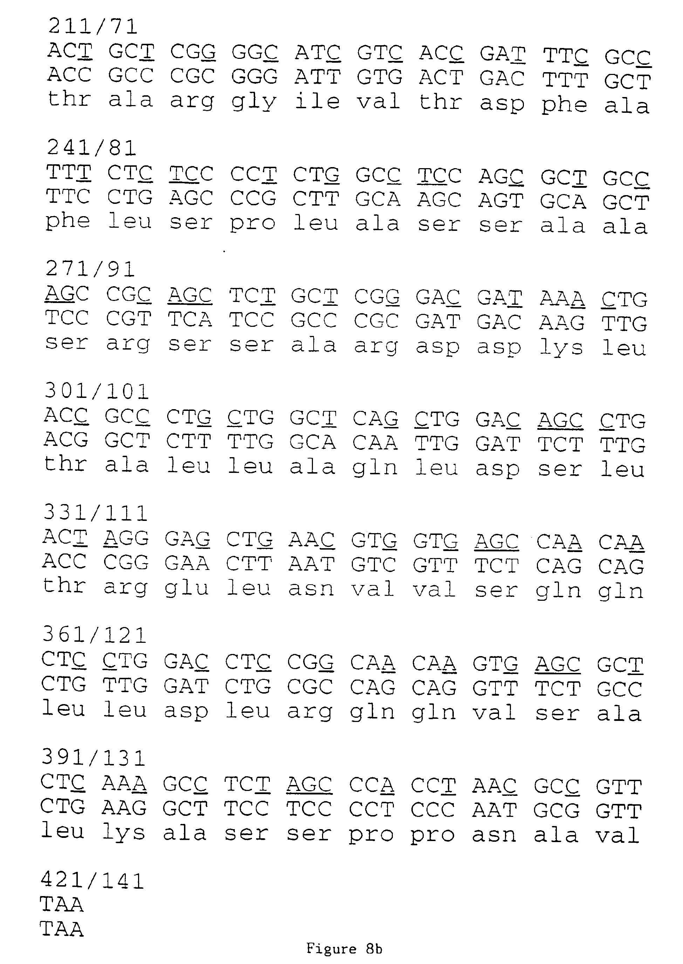

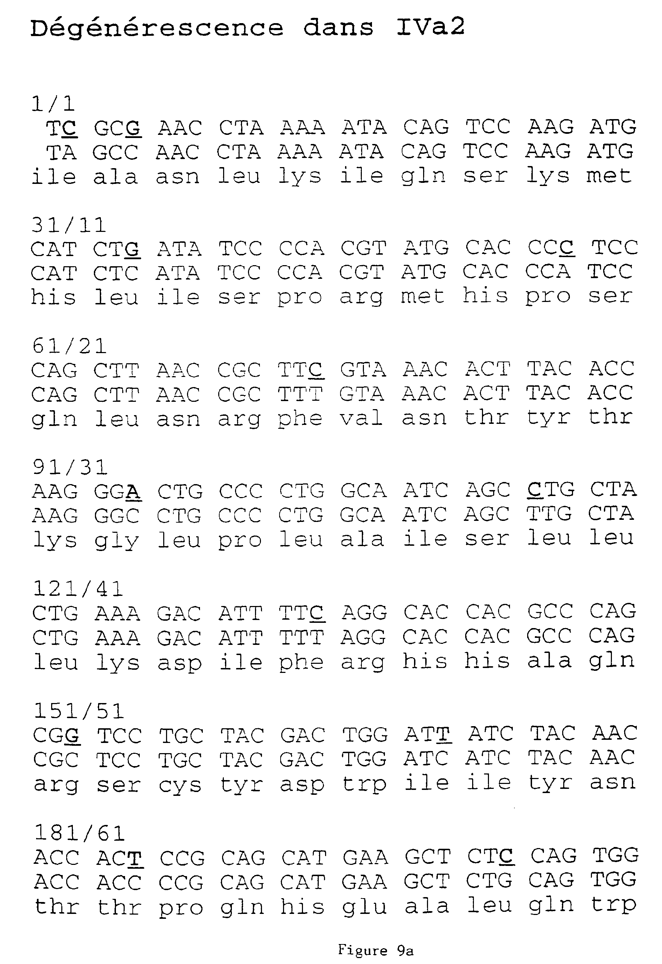

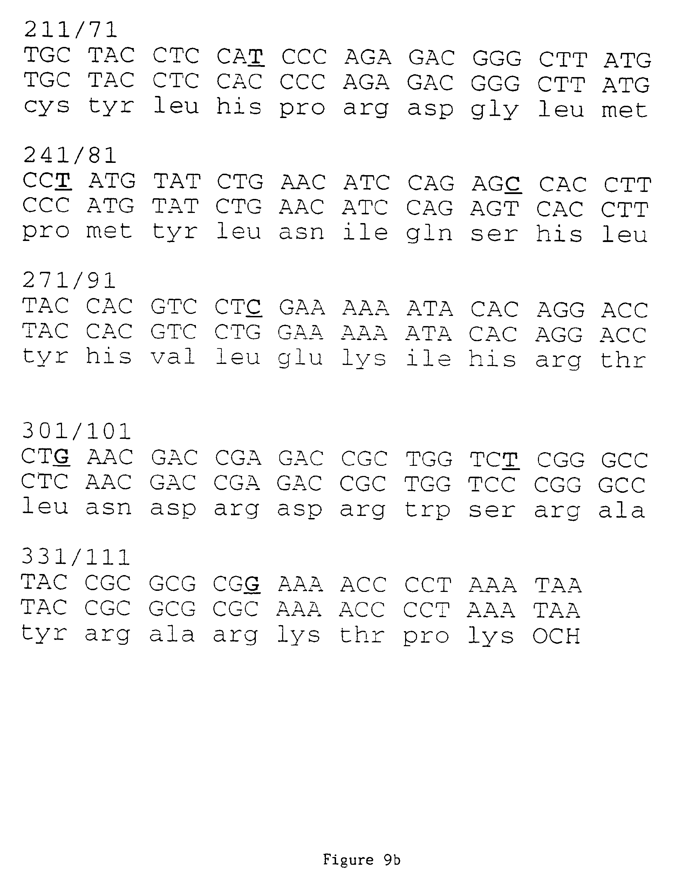

Dans un premier exemple précis de syngen (syngen # 1), les modifications suivantes ont été apportées:
- Dans le promoteur pIX : la séquence a été réduite autant que possible : le fragment retenu ne contient que 56 nucléotides, au lieu de 135 nucléotides dans le vecteur originel. Une modification supplémentaire peut être apportée par modification des 9 nucléotides situés entre le site de liaison SP1 et la boite TATA.
- Dans le gène pIX : Toutes les positions ont été dégénérées, quand cela était possible, suivant la règle définie ci-dessus, sans modification de la séquence d'acides aminés résultante (SEQ ID N° 1). La comparaison de la séquence naturelle et de la séquence dégénérée ainsi que la struture du produit d'expression de ces séquence sont présentés dans la figure 8. Selon une autre stratégie, seulement une position (pb) sur 10 est dégénérée. Toujours selon une stratégie alternative, seulement une position sur 20.
- Dans la région intervalle entre pIX et IVa2 : la séquence de cette région, de 59 nucléotides, n'a pas été modifiée dans cet exemple.
- Dans le gène IVa2 : La séquence codante du gène IVa2 a été dégénérée selon la règle définie ci-dessus, toutes les 20 pb, sans modification de la séquence d'acides aminés résultante (voir SEQ ID N° 2). La séquence naturelle, la séquence dégénérée et la séquence d'acides aminés résultante sont présentées dans la figure 9. Selon une autre stratégie, une position (pb) sur 10 est dégénérée ou même toutes les positions, toujours sans modification de la séquence d'acides aminés résultante.

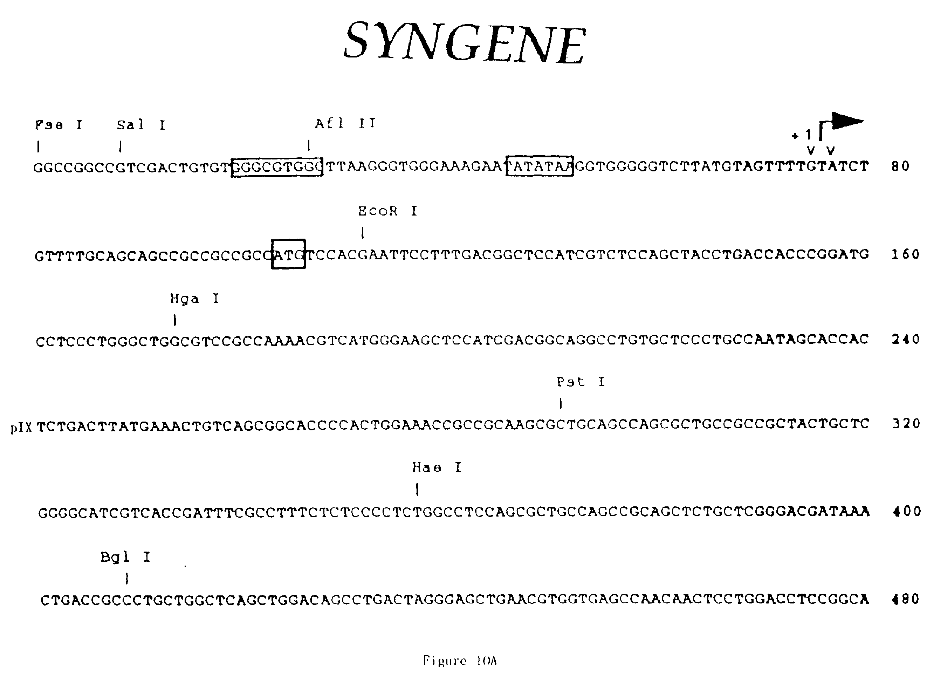

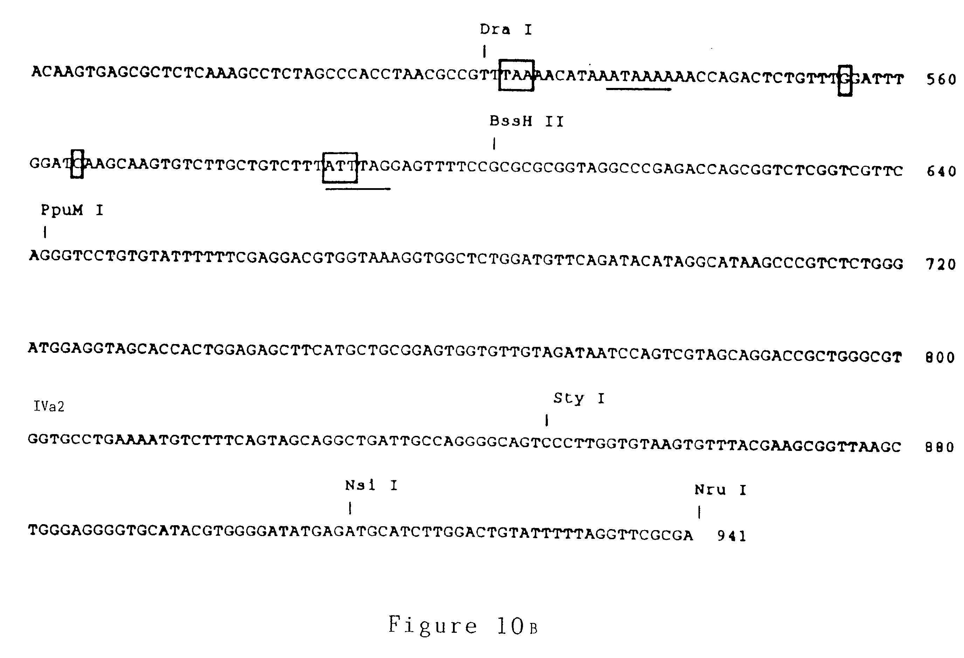

La structure du syngen # 1 est décrite schématiquement sur la figure 2. La séquence du syngen # 1 est représentée sur la figure 10 et par la séquence SEQ ID N° 3.

Un autre exemple de syngen est fourni par le syngen #2 dans lequel :
- la séquence du promoteur du gène pIX a été remplacée par une séquence modifiée telle que décrite en SEQ ID N° 6.
- la séquence du gène pIX a été remplacée selon la séquence décrite en SEQ ID N° 4 sans modification de la séquence d'acides aminés résultante.
- la séquence du gène IVa2 a été remplacée selon la séquence décrite en SEQ ID N° 5 sans modification de la séquence d'acides aminés résultante.
- La région de polyadénylation a été remplacée par la région qui remplit les mêmes fonctions au sein de l'adénovirus de type 7 (Ad 7) ; un adénovirus du sous groupe B qui est utilisé comme vecteur pour le transfert de gènes (Abrahamsen et al, 1997, J. Virol. 71,8946-8951). Cette séquence est décrite dans la séquence SEQ ID N°7.

L'ensemble de la séquence modifiée est appelée syngen #2 et est représenté par la séquence SEQ ID N°8.

### 3. Vérification de la séquence des syngen

Les caractéristiques de base des séquences naturelles et des syngen sont vérifiées par analyse informatique. Cette vérification a pour but de recherche la présence éventuelle, dans les syngen, de structures particulières, et notamment :
- de sites donneurs ou accepteurs d'épissage (programmes SpliceView et/ou Splice Net)
- de sites de polyadénylation (programme Hcpolya)
- de sites potentiels de liaison de facteurs transcriptionnels (programme Transfac)
- de régions susceptibles de former des structures secondaires particulières de type répétitions directes, épingle (programme Sigscan, BNL), y compris dans les ARNs correspondants (RNA folding)
- de sites consensus de type CHI ou M26.

Lorsque de telles séquence sont identifiées, les bases correspondantes peuvent éventuellement être remplacées selon la stratégie définie en 2. ci-avant.

### 4. Construction de virus défectifs portant le syngen # 1 en remplacement de la région naturelle correspondante

### 1. Matériel de départ :

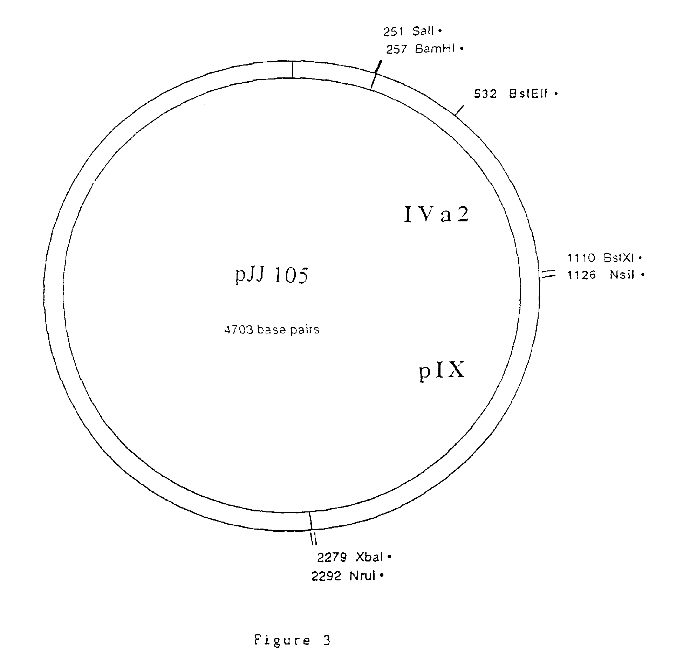

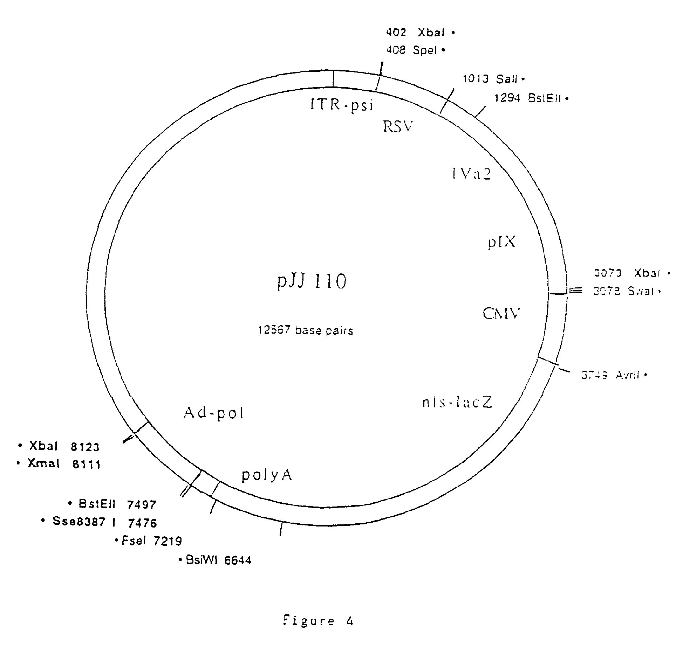

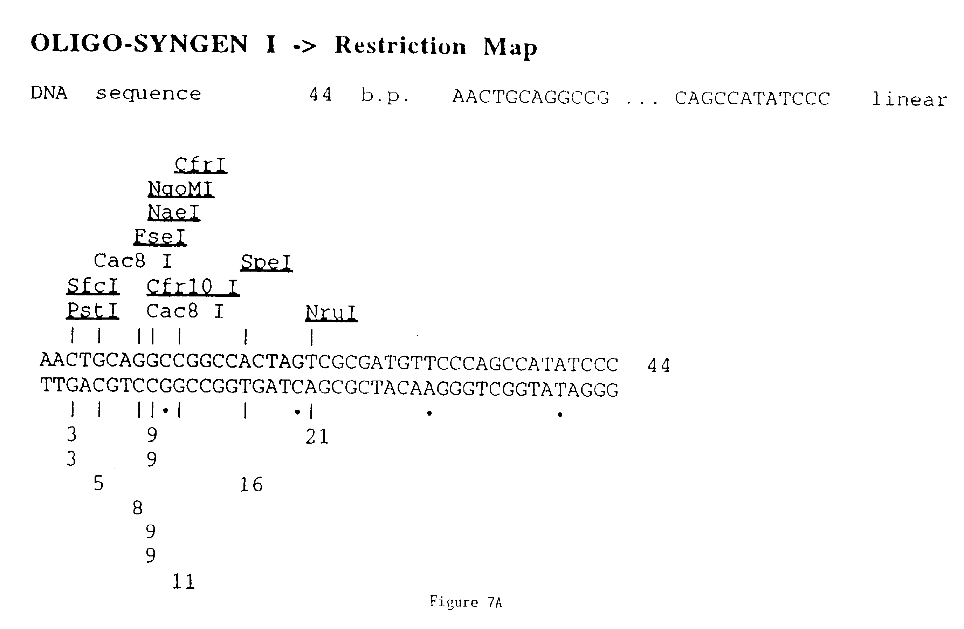

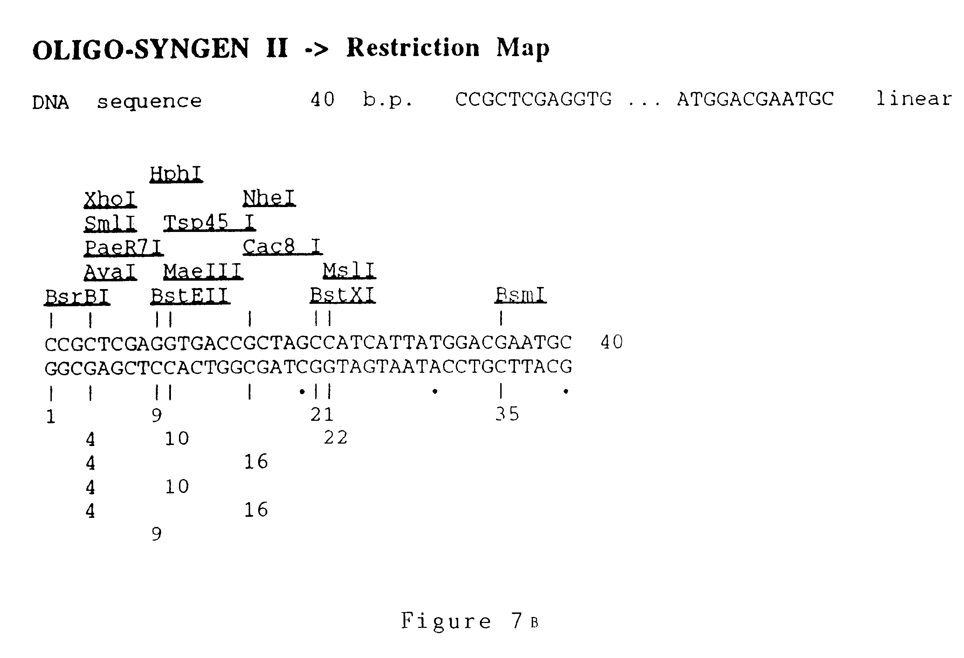

- Le syngen # 1 (SEQ ID N° 3)
- Les oligonucléotides Syngen I et Syngen II correspondant respectivement aux extrémités 5' et 3' du syngen # 1 sont données dans la figure 7.
- Plasmide pJJ105 (figure 3)
- Plasmide pJJ110 (figure 4)
- Plasmide pIC-20R (Invitrogen)
- Plasmide pSyngen : syngen cloné dans pBS (Stratagene)

### 2. Construction du génome viral défectif

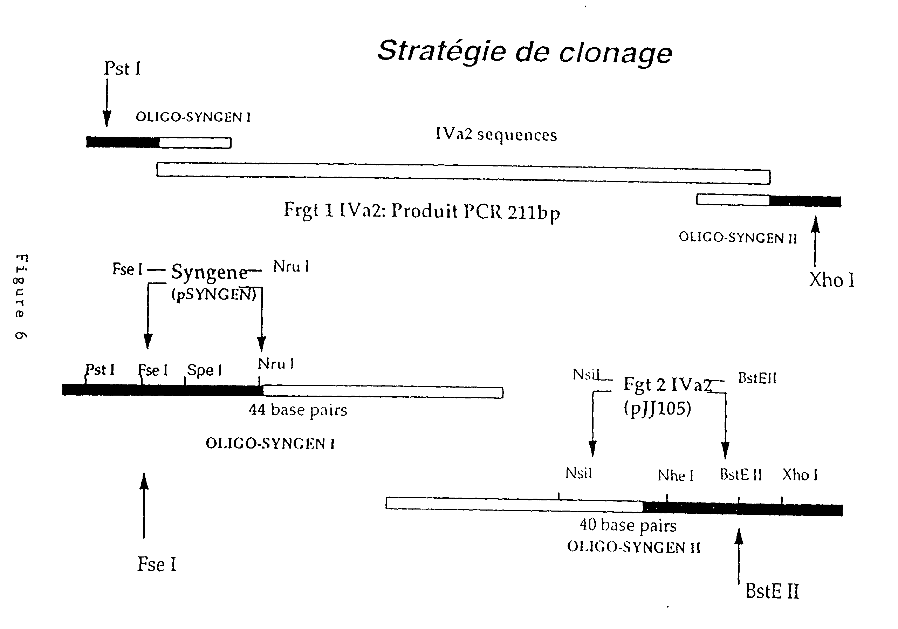

La stratégie de construction est décrite sur la figure 6.

Le plasmide pJJ110 est digéré par Xbal, et les fragments Xbal résultant de 4,8 et 5,0 kb sont ligaturés pour former le plasmide pJJ201. Le plasmide pJJ201 est ensuite digéré par BstEII et XmaI, rempli et ligaturé, pour former le plasmide pJJ202. Les deux sites BstEII et XmaI sont conservés.

Le produit d'amplification par PCR correspondant au fragment 1 du gène IVa2 (Fgt1 IVa2, figure 6) est généré en utilisant l'oligo syngen I et l'oligo syngen II et le plasmide pJJ105 comme matrice.
Séquence de l'oligo syngen I (SEQ ID N°9)
AACTGCAGGCCGGCCACTAGTCGCGATGTTCCCAGCCATATCCC
Séquence de l'oligo syngen II (SEQ ID N°10)
CCGCTCGAGGTGACCGCTAGCCATTATGGACGAATGC

Le fragment amplifié est inséré dans le site SmaI de pIC-20R pour générer pJJ203. Un fragment contigu du gène IVa2 du plasmide pJJ105 (Fragment 2 , identifié Fgt2 Iva2 dans la figure 6) est excisé par NsiI / BstEII puis inséré aux sites correspondants de pJJ203 pour générer pJJ204. Le syngen est ensuite digéré par FseI / NruI (figure 6) et inséré entre les sites correspondants de pJJ204 pour générer pJJ205.

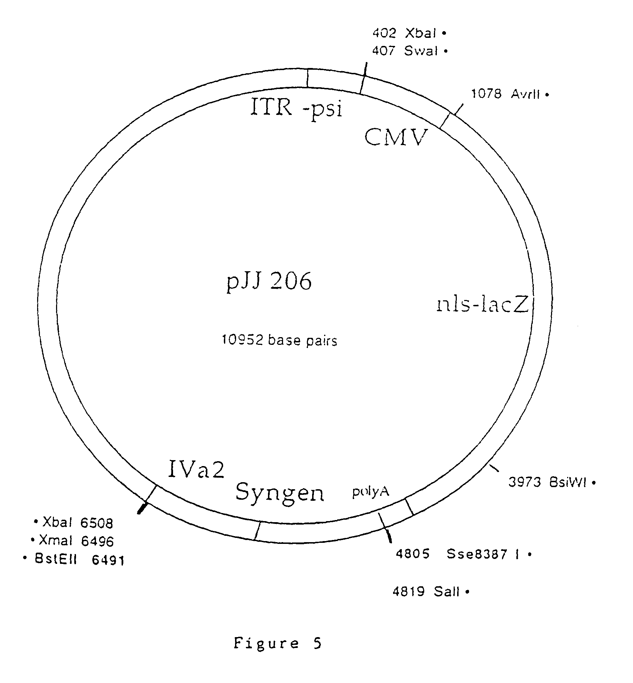

Le fragment complet (syngen#1-Fgt1+2 IVa2) de pJJ205 est digéré par Sse 83871 / BstEII et inséré aux sites correspondants dans pJJ202 pour générer pJJ206 (figure 5).

Ce fragment est ensuite utilisé pour préparer un plasmide procaryote comprenant un génome d'adénovirus modifié (défectif pour E1 et éventuellement E3) contenant le syngen # 1, selon la méthode décrite dans Crouzet et al (PNAS (94) 1414-1419 (1997)). Le plasmide obtenu est ensuite transfecté dans les cellules 293 pour produire les virus correspondants.

Une expérience a été menée pour comparer la capacité d'émergence des ACR (Adenovirus compétent pour la réplication) lors de la propagation à l'aide de la cellule 293 de deux virus AV1.7#1CMVp53 et l'Ad5CMVp53.

Un vecteur (AV1.7#1CMVp53) muni de la séquence SEQ ID n°3 (syngen # 1) et armé d'une cassette d'expression p53 (CMV-p53-polyASV40) a été construit selon Crouzet et al (PNAS (94) 1414-1419 (1997).

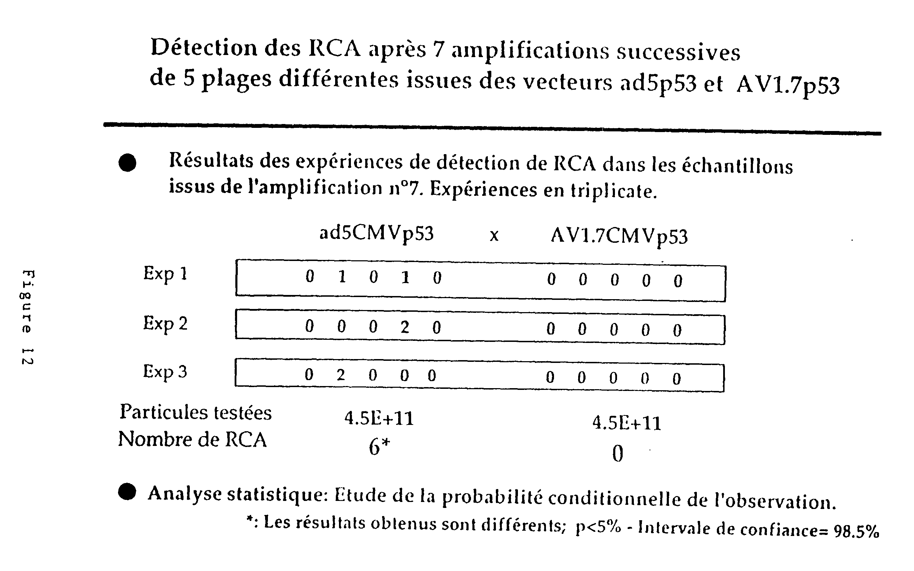

Ad5CMVp53 contient les régions de pIX-IVa2 de l'Ad5 (non modifiées). La cassette d'expression p53 est isogénique pour les deux virus. Les deux virus AV1.7#1CMVp53 et l'Ad5CMVp53 ont une productivité similaire dans la cellule 293. Pour chacun des virus, 5 plages ont été purifiées sur la cellule 293. Les 2x5 plages ont été amplifiés au cours de 7 passages successifs. A l'issue de cette amplifications, les 2x5 échantillons du passage 7 ont été analysé dans une expérience de détection des ACR. L'expérience a été effectuée en triplicate. Les résultats sont présentés dans la figure 12.

La méthode de détection des ACR est une méthode quantitative qui permet de compter des plages dont le nombre correspond au nombre de ACR dans la dose de l'échantillon testé. Une dose donnée de virus d'un échantillon (classiquement 3x10¹⁰ pv) est utilisée pour infecter un tapis cellulaire d'une cellule non-transcomplémentante (cad n'exprimant pas E1, en l'occurence la cellule A549) sur lequel est déposé une couche d'agar. Après 14 jours, on observe la formation de plage au sein du tapis lorsque la dose infectant de départ contient des ACR.

Les résultats de l'expérience de détection des ACR menée en triplicate sont : 0 RCA pour AV1.7#1CMVp53 et 6 RCA pour l'Ad5CMVp53. Ce résultat est statistiquement significatif lorsque l'on utilise une méthode statistique de comparaison faisant appel à la probabilité conditionnelle de l'observation. On observe une amélioration statistiquement significative en faveur du vecteur AV1.7#1CMVp53 en terme de réduction de l'émergence de ACR lors de la propagation dans la cellule 293 de ce vecteur comparé à un vecteur classique. L'ensemble de ces résultats permettent de montrer que la présence du syngen n'affecte pas les titres de virus produits, que les lots de virus comportant le syngen # 1 sont dépourvus de contamination par ACR.

### 5. Fonctionnalité de virus défectifs portant le syngen # 2 en remplacement de la région naturelle correspondante

De la même façon, il a été construit, un vecteur similaire avec le syngen # 2 et armé d'une cassette d'expression LacZ (CMV-LacZ) : AV 1.7 #2CMV lacZ. La productivité de ce vecteur a été comparée à celle d'un vecteur armé d'une cassette LacZ (RSV LacZ) dont les régions pIX et Iva2 n'ont pas été modifiées (séquence de l'Ad5 sauvage) : AV1.0 RSV lacZ. La productivité des deux vecteurs AV 1.7 #2CMV lacZ et AV1.0 RSV lacZ a été testée en parallèle dans des cellules 293. Les résultats sont présentés dans le tableau ci-dessous.

| | **AV 1.7 #2CMV lacZ** | **AV1.0 RSV lacZ** |
|---|---|---|
| Titre Stock 1 (pv/ml) | 1.12 10¹² | 1.50 10¹² |
| Titre Stock 2 (pv/ml) | 1.52 10¹² | 1.82 10¹² |
| Productivité (pv/cellule) testée sur Stock 2 | 10133 | 12133 |
| Productivité (tdu/cellule) testée sur Stock 2 | 203 | 207 |

Ces résultats montrent que le syngen #2 présentant une homologie plus réduite que le syngen #1 et qui contient, outre des mutations silencieuses au niveau des gène pIX et Iva2, des modifications au niveau des régions non codantes (i.e. au niveau du promoteur du gène pIX et des séquences de polyadénylation imbriquées de pIX et Iva2) ; un tel syngen, lorsqu'il est introduit en remplacement des séquences sauvages de l'adénovirus de type 5, s'avère être viable et assurer une productivité élevée.

### 6. Construction de virus défectifs dans lesquels seule la séquence naturelle du gène pIX est remplacée par la séquence dégénérée décrite en SEQ ID N°1 et la partie de la région homologue correspondant au gène IVa2 est déplacée dans la région E4.

Selon une autre variante de réalisation, seule la séquence naturelle du gène pIX est remplacée par la séquence dégénérée décrite en SEQ ID N°1 et la partie de la région homologue correspondant au gène IVa2 est déplacée dans la région E4. Le clonage du gène Iva2 a été décrit dans la demande WO96/10088 incluse ici par référence.

Un premier plasmide nommé pIE11 a été construit et contient les 3 régions suivantes clonées dans un plasmide colE1 porteur des gènes KanR et SacB :
- un produit de PCR fait sur l'Ad5 avec les amorces :
   5'-atcgatcgATAACAGTCAGCCTTACC-3' et
   5'-agctgaattcCATCATCAATAATATACC-3';
   ce produit de PCR contient l'ITR droite et le promoteur de E4 jusqu'à l'ATG d'orf1 non inclus (nucléotides 35525 à 35937)
- le cDNA du gène IVa2 décrit dans la demande de brevet WO/9610088.
- le fragment XcmI-EcoRV d'Ad5 contenant les séquences nécessaires pour l'épissage d'orf6 de E4 et la séquence codante d'orf6 jusqu'au site EcoRV.

Le cDNA de IVa2 dans le plasmide pIE11 est précédé d'un ATG excédentaire. Ce site est détruit sur le plasmide pIE11 par mutagénèse dirigée à l'aide d'un kit Transfomer Site-directed mutagenesis de Clontech pour former le plasmide pIE12 dans lequel cet ATG a été détruit.

Le plasmide pIE12c dérivé du plasmide pIE12 contient un génome d'adénovirus 5 modifié de la façon suivante: déplacement du gène de la IVa2 de son locus naturel vers la région E4, remplacement des orf1 à 4 de E4 au profit du cDNA de IVa2, délétion de E3, insertion d'une cassette RSV-lacZ en lieu et place de E1. Le plasmide pIE12c a été obtenu par recombinaison avec le plasmide pXL2788BGal tel que décrit dans la demande WO 96/10088 en utilisant la technologie décrite dans Crouzet et al (PNAS (94) 1414-1419 (1997)) incluse ici par référence.

Après digestion enzymatique par Pacl, le plasmide pIE12c a été transfecté dans 293 et le virus obtenu amplifié. On observe que le profil de restriction du virus AdIE12 est celui attendu.

Les analyses effectuées sur les lots de virus produits permettent de montrer que la présence de la séquence dégénérée du gène pIX en lieu et place de la séquence naturelle et le déplacement du gène IVa2 dans la région E4 n'affecte pas les titres de virus produits et que les lots de virus sont dépourvus de contamination par ACR lorsque ceux ci sont testés dans les conditions décrites ci-avant.

### REFERENCES BIBLIOGRAPHIQUES

Ahern et al (1991) PNAS 88:105
Aiello et al. (1979) Virol. 94:460
Babiss et Vales (1991) J. Virol. 65:598
Epstein (1991) J.Virol. 65:4475
Gangloff et al. (1994) Experientia 50:261
Holliday (1964) Genet. Res. 5 :282-304
Jeong-Yu et Carroll (1992) Mol. Cell Biol. 12(1) : 112-9
Lin et al. (1990) Mol. Cell Biol. 10(1) : 103-12
Matsui (1989) Mol. Cell. Biol. 9:4265
Meselson M. Radding C. (1975) Proc. Natl. Acad. Sci. USA, 80 : 358-361.
Spector (1983) Virol. 130:533
Szostak et al. (1983) Cell 33 : 25-35.
Waldman et Liskay (1987) PNAS 84(15) 5340-4
Weinberg (1986) J. Virol 57:833
Williams et Ustacelibi (1971) J. Gen. Virol. 13:345
Young (1995) Current Topics in Microbiol. and Immunol. 199:89

### LISTE DE SEQUENCES

(1) INFORMATIONS GENERALES:
   (i) DEPOSANT:
      (A) NOM: RHONE-POULENC RORER
      (B) RUE: 20, avenue Raymond Aron
      (C) VILLE: ANTONY
      (E) PAYS: FRANCE
      (F) CODE POSTAL: 92165
      (H) TELECOPIE: 01.55.71.72.91
   (ii) TITRE DE L'INVENTION: Methode de reduction des evenements de recombinaison homologue
   (iii) NOMBRE DE SEQUENCES:
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 424 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..424
   (xi) DESCRIPTION DE LA SEQUENCE: SEQ ID NO: 1:
   ATG TCC ACG AAT TCC TTT GAC GGC TCC ATC GTC TCC AGC TAC CTG ACC    48
   Met Ser Thr Asn Ser Phe Asp Gly Ser Ile Val Ser Ser Tyr Leu Thr
     1               5                  10                  15
   ACC CGG ATG CCT CCC TGG GCT GGC GTC CGC CAA AAC GTC ATG GGA AGC     96
   Thr Arg Met Pro Pro Trp Ala Gly Val Arg Gln Asn Val Met Gly Ser
                    20                  25                  30
   TCC ATC GAC GGC AGG CCT GTG CTC CCT GCC AAT AGC ACC ACT CTG ACT    144
   Ser Ile Asp Gly Arg Pro Val Leu Pro Ala Asn Ser Thr Thr Leu Thr
                35                  40                  45
   TAT GAA ACT GTC AGC GGC ACC CCA CTG GAA ACC GCC GCA AGC GCT GCA    192
   Tyr Glu Thr Val Ser Gly Thr Pro Leu Glu Thr Ala Ala Ser Ala Ala
        50                  55                  60
   GCC AGC GCT GCC GCC GCT ACT GCT CGG GGC ATC GTC ACC GAT TTC GCC    240
   Ala Ser Ala Ala Ala Ala Thr Ala Arg Gly Ile Val Thr Asp Phe Ala
    65                  70                  75                  80
   TTT CTC TCC CCT CTG GCC TCC AGC GCT GCC AGC CGC AGC TCT GCT CGG    288
   Phe Leu Ser Pro Leu Ala Ser Ser Ala Ala Ser Arg Ser Ser Ala Arg
                    85                  90                  95
   GAC GAT AAA CTG ACC GCC CTG CTG GCT CAG CTG GAC AGC CTG ACT AGG    336
   Asp Asp Lys Leu Thr Ala Leu Leu Ala Gln Leu Asp Ser Leu Thr Arg
               100                 105                 110
   GAG CTG AAC GTG GTG AGC CAA CAA CTC CTG GAC CTC CGG CAA CAA GTG    384
   Glu Leu Asn Val Val Ser Gln Gln Leu Leu Asp Leu Arg Gln Gln Val
           115                 120                 125
   AGC GCT CTC AAA GCC TCT AGC CCA CCT AAC GCC GTT TAA  A             424
   Ser Ala Leu Lys Ala Ser Ser Pro Pro Asn Ala Val  *
       130                 135                 140
(2) INFORMATIONS POUR LA SEQ ID NO: 2 :
   (i) CARACTERISTIQUES DE LA SEQUENCE:
      (A) LONGUEUR: 357 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..354
   (xi) DESCRIPTION DE LA SEQUENCE: SEQ ID NO: 2:
   ATC GCG AAC CTA AAA ATA CAG TCC AAG ATG CAT CTG ATA TCC CCA CGT     48
   Ile Ala Asn Leu Lys Ile Gln Ser lys Met His Leu Ile Ser Pro Arg
    1               5                  10                  15
   ATG CAC CCC TCC CAG CTT AAC CGC TTC GTA AAC ACT TAC ACC AAG GGA     96
   Met His Pro Ser Gln Leu Asn Arg Phe Val Asn Thr Tyr Thr Lys Gly
               20                  25                  30
   CTG CCC CTG GCA ATC AGC CTG CTA CTG AAA GAC ATT TTC AGG CAC CAC    144
   Leu Pro Leu Ala Ile Ser Leu Leu Leu Lys Asp Ile Phe Arg His His
           35                  40                  45
   GCC CAG CGG TCC TGC TAC GAC TGG ATT ATC TAC AAC ACC ACT CCG CAG    192
   Ala Gln Arg Ser Cys Tyr Asp Trp Ile Ile Tyr Asn Thr Thr Pro Gln
       50                  55                  60
   CAT GAA GCT CTC CAG TGG TGC TAC CTC CAT CCC AGA GAC GGG CTT ATG    240
   His Glu Ala Leu Gln Trp Cys Tyr Leu His Pro Arg Asp Gly Leu Met
    65                  70                  75                  80
   CCT ATG TAT CTG AAC ATC CAG AGC CAC CTT TAC CAC GTC CTC GAA AAA    288
   Pro Met Tyr Leu Asn Ile Gln Ser His Leu Tyr His Val Leu Glu Lys
                   85                  90                  95
   ATA CAC AGG ACC CTG AAC GAC CGA GAC CGC TGG TCT CGG GCC TAC CGC    336
   Ile His Arg Thr Leu Asn Asp Arg Asp Arg Trp Ser Arg Ala Tyr Arg
              100                 105                 110
   GCG CGG AAA ACC CCT AAA TAA                                        357
   Ala Arg Lys Thr Pro Lys
          115
(2) INFORMATIONS POUR LA SEQ ID NO: 3:
   (i) CARACTERISTIQUES DE LA SEQUENCE:
      (A) LONGUEUR: 941 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: exon
      (B) EMPLACEMENT : 1..941
   (xi) DESCRIPTION DE LA SEQUENCE: SEQ ID NO: 3:
   GGCCGGCCGT CGACTGTGTG GGCGTGGCTT AAGGGTGGGA AAGAATATAT AAGGTGGGGG   60
   TCTTATGTAG TTTTGTATCT GTTTTGCAGC AGCCGCCGCC GCCATGTCCA CGAATTCCTT  120
   TGACGGCTCC ATCGTCTCCA GCTACCTGAC CACCCGGATG CCTCCCTGGG CTGGCGTCCG  180
   CCAAAACGTC ATGGGAAGCT CCATCGACGG CAGGCCTGTG CTCCCTGCCA ATAGCACCAC  240
   TCTGACTTAT GAAACTGTCA GCGGCACCCC ACTGGAAACC GCCGCAAGCG CTGCAGCCAG  300
   CGCTGCCGCC GCTACTGCTC GGGGCATCGT CACCGATTTC GCCTTTCTCT CCCCTCTGGC  360
   CTCCAGCGCT GCCAGCCGCA GCTCTGCTCG GGACGATAAA CTGACCGCCC TGCTGGCTCA  420
   GCTGGACAGC CTGACTAGGG AGCTGAACGT GGTGAGCCAA CAACTCCTGG ACCTCCGGCA  480
   ACAAGTGAGC GCTCTCAAAG CCTCTAGCCC ACCTAACGCC GTTTAAAACA TAAATAAAAA  540
   ACCAGACTCT GTTTGGATTT GGATCAAGCA AGTGTCTTGC TGTCTTTATT TAGGAGTTTT  600
   CCGCGCCTGG TAGGCCCGAG ACCAGCGGTC TCGGTCGTTC AGGGTCCTGT GTATTTTTTC  660
   GAGGACGTGG TAAAGGTGGC TCTGGATGTT CAGATACATA GGCATAAGCC CGTCTCTGGG  720
   ATGGAGGTAG CACCACTGGA GAGCTTCATG CTGCGGAGTG GTGTTGTAGA TAATCCAGTC  780
   GTAGCAGGAC CGCTGGGCGT GGTGCCTGAA AATGTCTTTC AGTAGCAGGC TGATTGCCAG  840
   GGGCAGTCCC TTGGTGTAAG TGTTTACGAA GCGGTTAAGC TGGGAGGGGT GCATACGTGG  900
   GGATATGAGA TGCATCTTGG ACTGTATTTT TAGGTTCGCG A                      941
(2) INFORMATIONS POUR LA SEQ ID NO: 4:
   (i) CARACTERISTIQUES DE LA SEQUENCE:
      (A) LONGUEUR: 423 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT : 1..423
   (xi) DESCRIPTION DE LA SEQUENCE: SEQ ID NO: 4:
   ATG AGC ACG AAT TCG TTT GAC GGA AGC ATC GTC AGC TCA TAC TTG ACC      48
   Met Ser Thr Asn Ser Phe Asp Gly Ser Ile Val Ser Ser Tyr Leu Thr
    1               5                  10                  15
   ACG CGC ATG CCT CCC TGG GCC GGG GTG CGC CAG AAT GTC ATG GGC TCC      96
   Thr Arg Met Pro Pro Trp Ala Gly Val Arg Gln Asn Val Met Gly Ser
               20                  25                  30
   AGC ATT GAC GGT CGC CCT GTC CTG CCT GCA AAC TCT ACC ACT TTG ACC     144
   Ser Ile Asp Gly Arg Pro Val Leu Pro Ala Asn Ser Thr Thr Leu Thr
           35                  40                  45
   TAC GAA ACC GTG TCT GGC ACG CCG TTG GAA ACT GCA GCA TCC GCC GCC     192
   Tyr Glu Thr Val Ser Gly Thr Pro Leu Glu Thr Ala Ala Ser Ala Ala
       50                  55                  60
   GCT AGC GCC GCT GCA GCT ACC GCC CGC GGG ATC GTG ACT GAT TTT GCT     240
   Ala Ser Ala Ala Ala Ala Thr Ala Arg Gly Ile Val Thr Asp Phe Ala
   65                  70                  75                  80
   TTT CTG AGC CCG CTT GCC AGC AGT GCA GCC TCC CGT TCA TCT GCC CGC     288
   Phe Leu Ser Pro Leu Ala Ser Ser Ala Ala Ser Arg Ser Ser Ala Arg
                   85                  90                  95
   GAT GAC AAA TTG ACG GCT CTT CTG GCT CAG CTG GAT TCT TTG ACT CGG     336
   Asp Asp Lys Leu Thr Ala Leu Leu Ala Gln Leu Asp Ser Leu Thr Arg
               100                 105                 110
   GAA CTT AAC GTC GTT TCT CAG CAA CTG TTG GAC CTG CGC CAG CAA GTT     384
   Glu Leu Asn Val Val Ser Gln Gln Leu Leu Asp Leu Arg Gln Gln Val
           115                 120                 125
   TCT GCC CTC AAG GCT TCT TCC CCT CCC AAT GCC GTT TAA                 423
   Ser Ala Leu Lys Ala Ser Ser Pro Pro Asn Ala Val  *
       130                 135                 140
(2) INFORMATIONS POUR LA SEQ ID NO: 4:
   (i) CARACTERISTIQUES DE LA SEQUENCE:
      (A) LONGUEUR: 141 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Ser Thr Asn Ser Phe Asp Gly Ser Ile Val Ser Ser Tyr Leu Thr
     1               5                  10                  15
   Thr Arg Met Pro Pro Trp Ala Gly Val Arg Gln Asn Val Met Gly Ser
                20                  25                  30
   Ser Ile Asp Gly Arg Pro Val Leu Pro Ala Asn Ser Thr Thr Leu Thr
            35                  40                  45
   Tyr Glu Thr Val Ser Gly Thr Pro Leu Glu Thr Ala Ala Ser Ala Ala
        50                  55                  60
   Ala Ser Ala Ala Ala Ala Thr Ala Arg Gly Ile Val Thr Asp Phe Ala
    65                  70                  75                  80
   Phe Leu Ser Pro Leu Ala Ser Ser Ala Ala Ser Arg Ser Ser Ala Arg
                    85                  90                  95
   Asp Asp Lys Leu Thr Ala Leu Leu Ala Gln Leu Asp Ser Leu Thr Arg
               100                 105                 110
   Glu Leu Asn Val Val Ser Gln Gln Leu Leu Asp Leu Arg Gln Gln Val
           115                 120                 125
   Ser Ala Leu Lys Ala Ser Ser Pro Pro Asn Ala Val  *
       130                 135                 140
(2) INFORMATIONS POUR LA SEQ ID NO: 5:
   (i) CARACTERISTIQUES DE LA SEQUENCE:
      (A) LONGUEUR: 270 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT: 1..270
   (xi) DESCRIPTION DE LA SEQUENCE: SEQ ID NO: 5:
   ACC AAG GGC CTG CCC CTC GCA ATC AGC TTG CTA CTC AAA GAC ATT TTT      48
   Thr Lys Gly Leu Pro Leu Ala Ile Ser Leu Leu Leu Lys Asp Ile Phe
    1               5                  10                  15
   AGG CAT CAC GCC CAG CGC TCC TGT TAC GAC TGG ATC ATC TAC AAT ACC      96
   Arg His His Ala Gln Arg Ser Cys Tyr Asp Trp Ile Ile Tyr Asn Thr
               20                  25                  30
   ACC CCG CAG CAT GAA GCC CTG CAG TGG TGC TAC CTG CAC CCC AGA GAC     144
   Thr Pro Gln His Glu Ala Leu Gln Trp Cys Tyr Leu His Pro Arg Asp
           35                  40                  45
   GGG CTG ATG CCC ATG TAT CTG AAT ATC CAG AGT CAC CTT TAT CAC GTC     192
   Gly Leu Met Pro Met Tyr Leu Asn Ile Gln Ser His Leu Tyr His Val
       50                  55                  60
   CTG GAA AAA ATA CAT AGG ACC CTC AAC GAC CGA GAT CGC TGG TCC CGG     240
   Leu Glu Lys Ile His Arg Thr Leu Asn Asp Arg Asp Arg Trp Ser Arg
    65                  70                  75                  80
   GCC TAT CGC GCG CGC AAA ACC CCT AAA TAA                              270
   Ala Tyr Arg Ala Arg Lys Thr Pro Lys  *
                    85                  90
(2) INFORMATIONS POUR LA SEQ ID NO: 5:
   (i) CARACTERISTIQUES DE LA SEQUENCE:
      (A) LONGUEUR: 90 acides aminés
      (B) TYPE: acide aminé
      (D) CONFIGURATION: linéaire
   (ii) TYPE DE MOLECULE: prot,ine
   (xi) DESCRIPTION DE LA SEQUENCE: SEQ ID NO: 5:
   Thr Lys Gly Leu Pro Leu Ala Ile Ser Leu Leu Leu Lys Asp Ile Phe
    1               5                  10                  15
   Arg His His Ala Gln Arg Ser Cys Tyr Asp Trp Ile Ile Tyr Asn Thr
               20                  25                  30
   Thr Pro Gln His Glu Ala Leu Gln Trp Cys Tyr Leu His Pro Arg Asp
           35                  40                  45
   Gly Leu Met Pro Met Tyr Leu Asn Ile Gln Ser His Leu Tyr His Val
       50                  55                  60
   Leu Glu Lys Ile His Arg Thr Leu Asn Asp Arg Asp Arg Trp Ser Arg
    65                  70                  75                  80
   Ala Tyr Arg Ala Arg Lys Thr Pro Lys  *
                    85                  90
(2) INFORMATIONS POUR LA SEQ ID NO: 6:
   (i) CARACTERISTIQUES DE LA SEQUENCE:
      (A) LONGUEUR: 103 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (ix) CARACTERISTIQUE:
      (A) NOM/CLE: -
      (B) EMPLACEMENT: 1..103
   (xi) DESCRIPTION DE LA SEQUENCE: SEQ ID NO: 6:
   GGCCGGCCGT CGACTGTGTG GGCGTGGCAT AAGGGTGGGA AAGAATATAT AAGGTCGGGG   60
   TCTCATCTAG TCTTGTATCT GATTTGCAGT AGCCGCCGCC ACC                    103
(2) INFORMATIONS POUR LA SEQ ID NO: 7:
   (i) CARACTERISTIQUES DE LA SEQUENCE:
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: -
      (B) EMPLACEMENT: 1 .. 52
   (xi) DESCRIPTION DE LA SEQUENCE: SEQ ID NO: 7:
      AGATCTCAAA TCAATAAATA AACAAATACT TGTTATAAAA ACAAATGAAT GT 52
(2) INFORMATIONS POUR LA SEQ ID NO: 8:
   (i) CARACTERISTIQUES DE LA SEQUENCE:
      (A) LONGUEUR: 847 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (ix) CARACTERISTIQUE:
      (A) NOM/CLE: -
      (B) EMPLACEMENT : 1..847
   (xi) DESCRIPTION DE LA SEQUENCE: SEQ ID NO: 8:
   GGCCGGCCGT CGACTGTGTG GGCGTGGCAT AAGGGTGGGA AAGAATATAT AAGGTCGGGG   60
   TCTCATCTAG TCTTGTATCT GATTTGCAGT AGCCGCCGCC ACCATGAGCA CGAATTCGTT  120
   TGACGGAAGC ATCGTCAGCT CATACTTGAC CACGCGCATG CCTCCCTGGG CCGGGGTGCG  180
   CCAGAATGTC ATGGGCTCCA GCATTGACGG TCGCCCTGTC CTGCCTGCAA ACTCTACCAC  240
   TTTGACCTAC GAAACCGTGT CTGGCACGCC GTTGGAAACT GCAGCATCCG CCGCCGCTAG  300
   CGCCGCTGCA GCTACCGCCC GCGGGATCGT GACTGATTTT GCTTTTCTGA GCCCGCTTGC  360
   CAGCAGTGCA GCCTCCCGTT CATCTGCCCG CGATGACAAA TTGACGGCTC TTCTGGCTCA  420
   GCTGGATTCT TTGACTCGGG AACTTAACGT CGTTTCTCAG CAACTGTTGG ACCTGCGCCA  480
   GCAAGTTTCT GCCCTCAAGG CTTCTTCCCC TCCCAATGCC GTTTAAAGAT CTCAAATCAA  540
   TAAATAAAGA AATACTTGTT ATAAAAACAA ATGAATGTTT ATTTAGGGGT TTTGCGCGCG  600
   CGATAGGCCC GGGACCAGCG ATCTCGGTCG TTGAGGGTCC TATGTATTTT TTCCAGGACG  660
   TGATAAAGGT GACTCTGGAT ATTCAGATAC ATGGGCATCA GCCCGTCTCT GGGGTGCAGG  720
   TAGCACCACT GCAGGGCTTC ATGCTGCGGG GTGGTATTGT AGATGATCCA GTCGTAACAG  780
   GAGCGCTGGG CGTGATGCCT AAAAATGTCT TTGAGTAGCA AGCTGATTGC GAGGGGCAGG  840
   CCCTTGG                                                            847
(2) INFORMATIONS POUR LA SEQ ID NO: 9:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (ix) CARACTERISTIQUE:
      (A) NOM/CLE: -
      (B) EMPLACEMENT : 1..4
   (xi) DESCRIPTION DE LA SEQUENCE: SEQ ID NO: 9:
      AACTGCAGGCCGGCCACTAGTCGCGATGTTCCCAGCCATATCCC
(2) INFORMATIONS POUR LA SEQ ID PIO: 10:
   (i) CARACTERISTIQUES DE LA SEQUENCE:
      (A) LONGUEUR: 37 paires de bases
      (B) TYPE: nucléotide
      (C) NOMBRE DE BRINS: simple
      (D) CONFIGURATION: linéaire
   (ii) TYPE DE MOLECULE: Autre acide nucléique
   (iii) HYPOTHETIQUE: NON
   (iv) ANTI-SENS: NON
   (ix) CARACTERISTIQUE:
      (A) NOM/CLE: -
      (B) EMPLACEMENT: 1..37
   (xi) DESCRIPTION DE Lt1 SEQUENCE: SEQ ID NO: 10:
      CCGCTCGAGGTGACCGCTAGCCATTATGGACGAATGC

## Revendications

1. Procédé permettant de réduire la fréquence des événements de recombinaison homologue intra- ou intermoléculaire entre l'acide nucléique chromosomique de la cellule comprenant la région E1 d'un génome d'adénovirus ainsi qu'une région flanquante, et un acide nucléique extra-chromosomique comprenant un génome d'adénovirus défectif pour la région E1, **caractérisé en ce que** la séquence de l'un au moins des deux acides nucléiques est une séquence codante et est dégénérée.

2. Procédé selon la revendication 1 **caractérisé en ce que** la séquence est dégénérée à raison de 1 paire de bases au moins toutes les 20 paires de bases.

3. Procédé selon la revendication 1 **caractérisé en ce que** la séquence est dégénérée à raison de 1 paire de bases au moins toutes les 10 paires de bases.

4. Procédé selon la revendication 1 **caractérisé en ce que** la séquence est dégénérée sur toutes les positions possibles.

5. Procédé selon la revendication 1 **caractérisé en ce que** la dégénérescence est réalisée en fonction de l'usage des codons de la cellule de complémentation.

6. Procédé selon la revendication 1 **caractérisé en ce que** la séquence du génome d'adénovirus défectif pour la région E1 est dégénérée au niveau du gène pIX.

7. Procédé selon la revendication 6 **caractérisé en ce que** la séquence du génome d'adénovirus défectif pour la région E1 est dégénérée en outre au niveau du gène IVa2.

8. Procédé de préparation d'adénovirus recombinants défectifs par introduction dans une cellule de la lignée 293, ou dérivée de ladite lignée 293, du génome dudit adénovirus recombinant défectif, **caractérisé en ce que** ledit génome porte :
- une délétion de la région E1
- une dégénérescence dans le gène pIX ou dans les gènes pIX et IVa2.

9. Adénovirus recombinant défectif pour la région E1 dont le génome comprend au moins une région codante, **caractérisé en ce que** :
- la séquence de ladite région est dégénérée et
- la dégénérescence de ladite séquence permette de réduire la fréquence de recombinaison homologue intra- ou intermoléculaire entre l'acide nucléique chromosomique d'une cellule comprenant la région E1 d'un génome d'adénovirus ainsi qu'une région flanquante, et l'acide nucléique dudit adénovirus recombinant défectif pour la région E1.

10. Adénovirus selon la revendication 9 **caractérisé en ce que** la région dégénérée est une région codante et comprend le gène pIX.

11. Adénovirus selon la revendication 10 **caractérisé en ce que** la séquence dégénérée est la séquence SEQ ID N°1.

12. Adénovirus selon les revendications 10 ou 11 **caractérisé en ce qu'**en outre le gène IVa2 est présent dans une position génomique autre que sa position d'origine.

13. Adénovirus selon la revendication 12 **caractérisé en ce que** le gène IVa2 est positionné au niveau de la région E4.

14. Adénovirus selon la revendication 9 **caractérisé en ce que** la région dégénérée comprend les gènes pIX et IVa2.

15. Adénovirus selon la revendication 14 **caractérisé en ce que** la région dégénérée est la séquence SEQ ID N°3.

16. Adénovirus selon l'une des revendications 10-15 **caractérisé en ce qu'**il comporte une délétion de la région E1.

17. Adénovirus selon l'une des revendications 10 et 11 **caractérisé en ce que** la séquence dégénérée du gène pIX est la séquence SEQ ID N°1.

18. Cellule de la lignée 293, ou dérivée de la lignée 293, comprenant un adénovirus défectif pour la région E1 **caractérisée en ce que** la séquence de l'acide nucléique chromosomique de la cellule, comprenant la région E1 ainsi qu'une région flanquante dudit adénovirus défectif, ou la séquence de l'acide nucléique extra-chromosomique comprenant le génome dudit adénovirus, est une séquence codante et est dégénérée.

## Claims

1. Method for reducing the frequency of intra- or intermolecular homologous recombination events between the chromosomal nucleic acid of the cell comprising the E1 region of an adenovirus genome and also a flanking region, and an extrachromosomal nucleic acid comprising a genome of an adenovirus which is defective for the E1 region, **characterized in that** the sequence of at least one of the two nucleic acids is a coding sequence and is degenerated.

2. Method according to Claim 1, **characterized in that** the sequence is degenerated in a proportion of 1 base pair at least every 20 base pairs.

3. Method according to Claim 1, **characterized in that** the sequence is degenerated in a proportion of 1 base pair at least every 10 base pairs.

4. Method according to Claim 1, **characterized in that** the sequence is degenerated over all the possible positions.

5. Method according to Claim 1, **characterized in that** the degeneracy is produced as a function of the codon use of the complementing cell.

6. Method according to Claim 1, **characterized in that** the sequence of the genome of the said adenovirus defective for the E1 region is degenerated in the pIX gene.

7. Method according to Claim 6, **characterized in that** the sequence of the genome of the said adenovirus defective for the E1 region is in addition degenerated in the IVa2 gene.

8. Method for preparing defective recombinant adenoviruses by introducing, into a cell of the 293 line, or derived from the said 293 line, the genome of the said defective recombinant adenovirus, **characterized in that** the said genome carries:
- a deletion of the E1 region
- a degeneracy in the pIX gene or in the pIX and IVa2 genes.

9. Recombinant adenovirus which is defective for the E1 region, whose genome comprises at least one coding region, **characterized in that**:
- the sequence of the said region is degenerated, and
- the degeneracy of the said sequence makes it possible to reduce the frequency of intramolecular and intermolecular homologous recombination between the chromosomal nucleic acid of a cell comprising the E1 region of an adenovirus genome and also a flanking region, and the nucleic acid of the said recombinant adenovirus which is defective for the E1 region.

10. Adenovirus according to Claim 9, **characterized in that** the degenerate region is a coding region and comprises the pIX gene.

11. Adenovirus according to Claim 10, **characterized in that** the degenerate sequence is the sequence SEQ ID No. 1.

12. Adenovirus according to Claims 10 or 11, **characterized in that**, in addition, the IVa2 gene is present in a genomic position other than its original position.

13. Adenovirus according to Claim 12, **characterized in that** the IVa2 gene is positioned in the E4 region.

14. Adenovirus according to Claim 9, **characterized in that** the degenerate region comprises the pIX and IVa2 genes.

15. Adenovirus according to Claim 14, **characterized in that** the degenerate region is the sequence SEQ ID No. 3.

16. Adenovirus according to one of Claims 10 to 15, **characterized in that** it comprises a deletion of the E1 region.

17. Adenovirus according to either of Claims 10 and 11, **characterized in that** the degenerate sequence of the pIX gene is the sequence SEQ ID No. 1.

18. Cell of the 293 line, or derived from the 293 line, comprising an adenovirus which is defective for the E1 region, **characterized in that** the sequence of the chromosomal nucleic acid of the cell, comprising the E1 region and also a flanking region of the said defective adenovirus, or the sequence of the extrachromosomal nucleic acid which comprises the genome of the said adenovirus, is a coding sequence and is degenerated.

## Patentansprüche

1. Verfahren, das es ermöglicht, die Frequenz von intra- oder intermolekularen homologen Rekombinationsereignissen zwischen der chromosomalen Nukleinsäure der Zelle umfassend die E1-Region eines Adenovirus-Genoms sowie eine flankierende Region und einer extrachromosomalen Nukleinsäure umfassend ein für die E1-Region defizientes Adenovirusgenom zu reduzieren, **dadurch gekennzeichnet, daß** es sich bei der Sequenz von mindestens einer der beiden Nukleinsäuren um eine Codiersequenz handelt, die degeneriert ist.

2. Verfahren nach Anspruch 1, **dadurch gekennzeichnet, daß** die Sequenz im Ausmaß von mindestens einem Basenpaar pro 20 Basenpaare degeneriert ist.

3. Verfahren nach Anspruch 1, **dadurch gekennzeichnet, daß** die Sequenz im Ausmaß von mindestens einem Basenpaar pro 10 Basenpaare degeneriert ist.

4. Verfahren nach Anspruch 1, **dadurch gekennzeichnet, daß** die Sequenz an allen möglichen Positionen degeneriert ist.

5. Verfahren nach Anspruch 1, **dadurch gekennzeichnet, daß** die Degeneration in Abhängigkeit von "Codon Usage" der Komplementärzelle erfolgt.

6. Verfahren nach Anspruch 1, **dadurch gekennzeichnet, daß** die Sequenz des für die E1-Region defizienten Adenovirusgenoms am pIX-Gen degeneriert ist.

7. Verfahren nach Anspruch 6, **dadurch gekennzeichnet, daß** die Sequenz des für die E1-Region defizienten Adenovirusgenoms weiterhin am IVa2-Gen degeneriert ist.

8. Verfahren zur Herstellung von defizienten rekombinanten Adenoviren durch Einführung des Genoms des genannten defizienten rekombinanten Adenovirus in eine Zelle der Linie 293 oder in eine Zelle, die sich von dieser Linie 293 ableitet, **dadurch gekennzeichnet, daß** dieses Genom folgendes trägt:
- eine Deletion der E1-Region
- eine Degeneration im pIX-Gen oder im pIX- und im IVa2-Gen.

9. Für die E1-Region defizientes rekombinantes Adenovirus, dessen Genom mindestens eine Codierregion umfaßt, **dadurch gekennzeichnet, daß**:
- die Sequenz dieser Region degeneriert ist und
- es die Degeneration dieser Sequenz ermöglicht, die intra- oder intermolekulare homologe Rekombinationsfrequenz zwischen der chromosomalen Nukleinsäure einer Zelle umfassend die E1-Region eines Adenovirusgenoms sowie eine flankierende Region und der Nukleinsäure des genannten für die E1-Region defizienten rekombinanten Adenovirus zu reduzieren.

10. Adenovirus nach Anspruch 9, **dadurch gekennzeichnet, daß** es sich bei der degenerierten Region um eine Codierregion handelt, die das pIX-Gen umfaßt.

11. Adenovirus nach Anspruch 10, **dadurch gekennzeichnet, daß** es sich bei der degenerierten Sequenz um die Sequenz SEQ ID NR.1 handelt.

12. Adenovirus nach Anspruch 10 oder 11, **dadurch gekennzeichnet, daß** weiterhin das IVa2-Gen in einer genomischen Position vorliegt, bei der es sich nicht um seine ursprüngliche Position handelt.

13. Adenovirus nach Anspruch 12, **dadurch gekennzeichnet, daß** das IVa2-Gen in der E4-Region liegt.

14. Adenovirus nach Anspruch 9, **dadurch gekennzeichnet, daß** die degenerierte Region das pIX- und das IVa2-Gen umfaßt.

15. Adenovirus nach Anspruch 14, **dadurch gekennzeichnet, daß** es sich bei der degenerierten Region um die Sequenz SEQ ID NR. 3 handelt.

16. Adenovirus nach einem der Ansprüche 10-15, **dadurch gekennzeichnet, daß** es eine Deletion der E1-Region umfaßt.

17. Adenovirus nach einem der Ansprüche 10 und 11, **dadurch gekennzeichnet, daß** es sich bei der degenerierten Sequenz des pIX-Gens um die Sequenz SEQ ID Nr. 1 handelt.

18. Zelle der Linie 293, oder Zelle, die sich von der Linie 293 ableitet, umfassend ein für die E1-Region defizientes Adenovirus, **dadurch gekennzeichnet, daß** es sich bei der Sequenz der chromosomalen Nukleinsäure der Zelle, die die E1-Region sowie eine flankierende Region des genannten defizienten Adenovirus umfaßt, oder bei der Sequenz der extrachromosomalen Nukleinsäure, die das Genom des genannten Adenovirus umfaßt, um eine Codiersequenz handelt, die degeneriert ist.
